# EUROPÄISCHE PATENTSCHRIFT

(11) **EP 3 558 053 B1**
(45) Veröffentlichungstag und Bekanntmachung des Hinweises auf die Patenterteilung: **06.03.2024**
(21) Anmeldenummer: 17835635.8
(22) Anmeldetag: 15.12.2017
(51) Int. Cl.: A45D 44/00, A46B 5/00, A61B 5/00, A45D 24/00, A46B 9/02, A46B 15/00, G01N 29/14, G01N 29/44, G01N 29/46

(54) **HAARINFORMATIONS-ERMITTLUNGSVORRICHTUNG, HAARINFORMATIONS-ERMITTLUNGSSYSTEM UND VERFAHREN ZUM BEREITSTELLEN EINER HAARZUSTANDSINFORMATION**
HAIR INFORMATION COLLECTION DEVICE, HAIR INFORMATION COLLECTION SYSTEM, AND METHOD FOR PROVIDING HAIR CONDITION INFORMATION
DISPOSITIF DE DÉTERMINATION D'INFORMATIONS CAPILLAIRES, SYSTÈME DE DÉTERMINATION D'INFORMATIONS CAPILLAIRES ET PROCÉDÉ DE MISE À DISPOSITION D'UNE INFORMATION D'ÉTAT CAPILLAIRE

(30) Priorität: 22.12.2016 DE 102016225964
(43) Veröffentlichungstag der Anmeldung: 30.10.2019
(73) Patentinhaber: Henkel AG & Co. KGaA, 40589 Düsseldorf (DE)
(72) Erfinder: KATZAROV, Jordan, 40591 Düsseldorf (DE); LECHNER, Torsten, 40764 Langenfeld (DE)
(86) Internationale Anmeldenummer: PCT/EP2017/083148
(87) Internationale Veröffentlichungsnummer: WO 2018/114717

(56) Entgegenhaltungen:
- US-A1- 2015 342 515

## Beschreibung

Die Erfindung betrifft eine Haarinformations-Ermittlungsvorrichtung nach Anspruch 1, ein Haarinformations-Ermittlungssystem nach Anspruch 10 und ein Verfahren zum Bereitstellen einer Information, welche einen Zustand von Haaren eines Nutzers betrifft, nach Anspruch 11.

Nutzer (auch als Verbraucher bezeichnet) wissen oft nicht in welchem Maße ihre Haare geschädigt sind. Diese Information kann wichtig sein, damit ein/e richtige/s, d.h. zum Haarschädigungsgrad passende/s Haarpflegeprodukt und/oder Haarfärbemittel (auch als Haarcoloration bezeichnet) ausgewählt werden kann/können.

Insbesondere vermissen Verbraucher oft Produktempfehlungen in Bezug auf ihre äußere Haarschädigung (Schädigung der Haarkutikula (auch als Cuticula bezeichnet)), also beispielsweise bzgl. Rauheit, Frizz, Spliss, usw.

Die optimale Produktempfehlung verleiht geschädigtem Haar (Haar mit Schäden an der Cuticula) wieder zu Glätte und Glanz, wohingegen eine aufgrund fehlender Ausgangsdaten erfolgte kosmetische Falsch- oder Fehlbehandlung zeit- und kostenaufwändig sein kann.

Dokument US2015342515 A1 beschreibt eine digitale Bürste mit einem Bewegungssensor, einem Mikrofon und einer Kamera. Basierend auf den gemessenen Daten werden dem Nutzer Empfehlungen zur Haarpflege gegeben.

In verschiedenen Ausführungsbeispielen wird ein kosmetisches Verfahren bereitgestellt, bei dem äußere Haarschädigungen unter Betrachtung der Cuticula-Haaroberflächen mittels eines akustischen Emissionssystems analysiert werden können und ein Haarschädigungsgrad anhand von Geräuschen und Visualisierungen dargestellt werden kann.

Ferner kann das Verfahren in verschiedenen Ausführungsbeispielen als eine Grundlage für eine richtige, d.h. zum Haarschädigungsgrad passende, Produktempfehlung herangezogen werden. Beispielsweise kann der Nutzer eine exakte Produktempfehlung für eine Haarcoloration, Blondierung, Dauerwelle, Haarpflege und/oder ein Haarstyling passend zu seinem äußeren Haarschädigungsgrad erhalten.

In verschiedenen Ausführungsbeispielen kann ein Nutzer selbst seinen äußeren Haarschädigungsgrad ermitteln, beispielsweise ohne aufwändige Mikroskopie durchzuführen und/oder Hintergrundwissen zu einer entsprechenden Auswertung zu haben. Somit kann es dem Nutzer ermöglicht werden, bei einem Ermitteln seines Haarzustands auf fachkundige Hilfe zu verzichten.

In verschiedenen Ausführungsbeispielen kann die äußere Haarschädigung mittels einer Vorrichtung, die einen Sensor zum Erfassen akustischer Emissionen, z.B. ein Mikrofon, aufweist, z.B. mittels eines Kontaktmikrofonkammes, ermittelt werden. Die Vorrichtung wird auch als Haarinformations-Ermittlungsvorrichtung bezeichnet.

Hierin kann der Sensor zum Erfassen akustischer Emissionen der Einfachheit halber auch als Mikrofon bezeichnet werden. Sofern nicht anders angegeben und für die beschriebene Funktion geeignet, kann der Sensor zum Erfassen akustischer Emissionen allerdings auch beispielsweise ein Beschleunigungssensor (der geeignet sein kann, Beschleunigungen infolge akustischer Emissionen in einem gewissen Frequenzbereich zu erfassen) oder ähnliches sein.

Hierin kann Bezug genommen werden auf "die Sensoren", beispielsweise hinsichtlich einer Datenübertragung zwischen den Sensoren und einer Datenverarbeitungsvorrichtung, einer Anordnung der Sensoren, usw. Dies ist so zu verstehen, dass die Sensoren eine Gesamtheit von in der Haarinformations-Ermittlungsvorrichtung angeordneten Sensoren und/oder Sensorschaltkreisen aufweisen kann, z.B. eine Gesamtheit von Mikrofon/en, Geschwindigkeits-Sensorschaltkreis, ggf. Kamera, usw., oder, sofern dies aus dem Kontext hervorgeht, einen Teil der genannten Sensoren und/oder Sensorschaltkreise.

Die Haarinformations-Ermittlungsvorrichtung kann in verschiedenen Ausführungsbeispielen als Kontaktmikrofonkamm ausgeführt sein. Der Kontaktmikrofonkamm kann in verschiedenen Ausführungsbeispielen einen im Wesentlichen handelsüblichen Haarkamm aufweisen, an dem ein oder mehrere von außen angebrachte Messsysteme für akustische Emissionen (d.h. Schallemissionen; als ein beispielhaftes Messsystem für akustische Emissionen kann ein Kontaktmikrofon der Fa. Korg genutzt werden) und/oder Messsonden für akustische Emissionen angeschlossen sein.

Die Haarinformations-Ermittlungsvorrichtung kann in verschiedenen Ausführungsbeispielen ein oder mehrere Messsysteme für akustische Emissionen und/oder Sonden aufweisen, die fest in die Haarinformations-Ermittlungsvorrichtung, z.B. einen Haarkamm, eingebaut sind.

In verschiedenen Ausführungsbeispielen können mittels der Messsysteme für akustische Emissionen Signale von erzeugtem Schall oder Vibration aufgenommen werden, die bei einem Durchkämmen von Haaren eines Nutzers entstehen.

Die Haarinformations-Ermittlungsvorrichtung kann in verschiedenen Ausführungsbeispielen ein oder mehrere Messsysteme für akustische Emissionen und/oder Sonden aufweisen, z.B. eines oder mehrere Kontaktmikrofone und/oder einen Beschleunigungssensor. Die Haarinformations-Ermittlungsvorrichtung kann in verschiedenen Ausführungsbeispielen ferner einen internen oder externen Verstärker aufweisen zum Verstärken von mittels des Messsystems für akustische Emissionen gemessenen Signalen.

In verschiedenen Ausführungsbeispielen kann eine Analyse einer Haarschädigung bereitgestellt werden mittels Digitalisierens einer Information von einem Mikrofon (und mindestens eines weiteren Sensors) und Bereitstellens dieser Daten auf einer Cloud-Plattform. Das Mikrofon kann in verschiedenen Ausführungsbeispielen Teil einer Haarinformations-Ermittlungsvorrichtung sein.

Die Information kann nach einem Verarbeiten bereitgestellt werden bzw. sein für ein Vergleichen mit bereits aufgezeichneten Beispielen (Referenzdaten). Für die Referenzdaten kann ein Haarschädigungsgrad bekannt sein, so dass als ein Ergebnis des Vergleichs beispielsweise der Haarschädigungsgrad der ähnlichsten Referenzdaten als Ergebnis in digitaler Form bereitgestellt, z.B. zurückübermittelt, werden kann.

Mittels Verwendens verschiedener Sensoren wie z.B. Linsen, Gyroskope und Beschleunigungsmesser, kann es möglich sein, eine Position der Haarinformations-Ermittlungsvorrichtung zu ermitteln, z.B. eine Position in einer Hand einer Person, um eine geeignete kosmetische Haarpflege (z.B. Produkte, Behandlungen) zu ermitteln und einer unnötigen Haarschädigung, z.B. Haarverlust, vorzubeugen.

Die Datenübermittlung vom Messsystem für akustische Emissionen, z.B. dem Kontaktmikrofon und/oder dem Beschleunigungssensor, zum Verstärker kann in verschiedenen Ausführungsbeispielen mittels Kabel oder über bekannte Datenfunkstandards (z.B. Bluetooth, WLAN, NFC usw.) erfolgen.

Der Verstärker kann in verschiedenen Ausführungsbeispielen ein Signalaufbereitungs- und Verstärkungssystem (auch als Signalkonditionierungssystem bezeichnet) aufweisen, das mit einem Datenerfassungssystem verbunden sein kann, welches wiederum seinerseits mit einem Ergebnisspeicher, einer Korrekturverarbeitung und einem Ausgabesystem verbunden sein kann.

Jede einzelne der genannten Komponenten (Signalaufbereitungs- und Verstärkungssystem aufweisen, Datenerfassungssystem, Ergebnisspeicher, Korrekturverarbeitung, Ausgabesystem) kann in verschiedenen Ausführungsbeispielen handelsüblich oder nach Maß gebaut sein, und das Messsystem für akustische Emissionen kann beliebig mit einer, allen oder manchen der oben beschriebenen Komponenten kombiniert werden.

In verschiedenen Ausführungsbeispielen können die genannten Komponenten (oder eine oder mehrere davon) in einer elektronischen Vorrichtung, z.B. einer mobilen elektronischen Vorrichtung (auch als Mobile Device bezeichnet), z.B. einem Smartphone oder einem Tablet, oder z.B. in einer sonstigen Datenverarbeitungsvorrichtung (z.B. einem PC) bereitgestellt sein, z.B. kombiniert/integriert. In verschiedenen Ausführungsbeispielen kann ferner eine (ggf. weitere) externe Datenverarbeitungsvorrichtung, z.B. mittels einer Cloud, genutzt werden für eine Signalauswertung, z.B. als eine Erweiterung der Signalauswertung. Dafür können in verschiedenen Ausführungsbeispielen die mittels der Sensoren erfassten Signale mit in einer Datenbank hinterlegten Signalen (auch als Vergleichssignale, Vergleichsdaten, Referenzsignale oder Referenzdaten bezeichnet) verglichen werden. In verschiedenen Ausführungsbeispielen kann anhand dessen der äußere Haarschädigungsgrad eingeordnet werden, beispielsweise indem den Vergleichssignalen Haarschädigungsgrade zugeordnet sind, und der Haarschädigungsgrad des dem gemessenen Signals ähnlichsten Vergleichssignals den gemessenen Haaren zugeordnet wird.

In verschiedenen Ausführungsbeispielen können eine oder mehrere der genannten Komponenten (Signalaufbereitungs- und Verstärkungssystem aufweisen, Datenerfassungssystem, Ergebnisspeicher, Korrekturverarbeitung, Ausgabesystem) Teil einer elektronischen Schaltkreisvorrichtung sein, welche Teil der Haarinformations-Ermittlungsvorrichtung sein kann, beispielsweise in oder an einem Vorrichtungskörper der Haarinformations-Ermittlungsvorrichtung angebracht sein kann.

In verschiedenen Ausführungsbeispielen kann, abhängig davon, welche Komponente Teil der elektronischen Schaltkreisvorrichtung ist, ein teil- oder vollständig verarbeitetes Signal mittels der Haarinformations-Ermittlungsvorrichtung bereitgestellt werden, beispielsweise ein verstärktes akustisches Signal, ein korrigiertes akustisches Signal, ein unter Berücksichtigung einer ermittelten Geschwindigkeit verarbeitetes akustisches Signal, ein mittels Vergleichens des akustischen Signals mit einer Datenbank ermittelter Haarschädigungsgrad, eine daraus (z.B. ebenfalls mittels der Datenbank) abgeleitete Empfehlung, z.B. eine Empfehlung für ein kosmetisches Produkt- und/oder eine kosmetische Haarbehandlung, usw.

Das Signalaufbereitungs- und Verstärkungssystem kann mit dem Messsystem für akustische Emissionen verbunden sein, um ein empfangenes Geräusch, z.B. einen empfangenen Ton, zu verstärken und/oder ein über den Beschleunigungssensor empfangenes Signal zu einem Ton zu manipulieren.

In verschiedenen Ausführungsbeispielen können Daten, die von dem einem oder den mehreren Verstärker/n empfangen werden, an das Datenerfassungssystem übertragen werden. Alternativ können die Daten auch direkt von dem Messsystem für akustische Emissionen an das Datenerfassungssystem übertragen werden.

In verschiedenen Ausführungsbeispielen kann die Haarinformations-Ermittlungsvorrichtung ferner einen Geschwindigkeitssensor-Schaltkreis aufweisen zum Erfassen einer Geschwindigkeit der Haarinformations-Ermittlungsvorrichtung. In verschiedenen Ausführungsbeispielen, z.B. in einem Fall, dass die Haarinformations-Ermittlungsvorrichtung mehrteilig ist und nur ein Teil (z.B. ein Vorrichtungskörper), der die Sensoren aufweist, entlang der Haare des Nutzers bewegt wird, kann der Geschwindigkeitssensor-Schaltkreis so eingerichtet sein, dass er die Geschwindigkeit dieses Teils erfasst. Anders ausgedrückt kann der Geschwindigkeitssensor-Schaltkreis so eingerichtet sein, dass er eine Geschwindigkeit erfasst, mit welcher derjenige Teil der Haarinformations-Ermittlungsvorrichtung, dessen Bewegung entlang der Haare des Nutzers zu einer akustischen Emission führt, bewegt wird oder sich bewegt.

Der Geschwindigkeitssensor-Schaltkreis kann in verschiedenen Ausführungsbeispielen mindestens einen Sensor aufweisen, der, (beispielsweise in einem Fall von nur einem Sensor) für sich allein genommen oder (beispielsweise bei mehreren Sensoren) basierend auf Sensorwerten von den mehreren Sensoren in Kombination, ein Ermitteln einer Geschwindigkeit, mit welcher der Geschwindigkeitssensor-Schaltkreis bewegt wird, ermöglicht.

In verschiedenen Ausführungsbeispielen kann der Geschwindigkeitssensor-Schaltkreis als den mindestens einen Sensor beispielsweise einen Beschleunigungssensor aufweisen, der beispielsweise als ein MEMS-Sensor oder als ein piezoelektrischer Sensor gebildet sein kann.

In verschiedenen Ausführungsbeispielen können die Daten, die mittels des Geschwindigkeitssensor-Schaltkreises erfasst werden, an das Datenerfassungssystem übertragen werden.

In verschiedenen Ausführungsbeispielen kann ein Verfahren zum Bereitstellen einer Information bezüglich Haaren eines Nutzers akustische Signale verwenden, die durch Kontakt zwischen einer Haarinformations-Ermittlungsvorrichtung und der Haaroberfläche und/oder zwischen einer ersten Haaroberfläche und einer zweiten Haaroberfläche durch ein Hinweggleiten erzeugt werden. Dadurch können unterschiedliche Schwingungsmuster zugänglich gemacht werden, die durch ein Messsystem für akustische Emissionen (z.B. ein Mikrofon) erfasst werden können. Die akustischen Emissionen, z.B. die unterschiedlichen Schwingungsmuster, können beispielsweise mittels einen Ergebnisspeicher, eine Korrekturverarbeitung und ein Ausgabesystem aufgezeichnet werden.

Es wurde festgestellt, dass eine Amplitude einer Schwingung und von Vibrationen und ein Frequenzgehalt eines akustischen Signals oder von Wellen (z.B. von Vibrationen), die als Funktion der Zeit überwacht werden, sehr empfindlich gegenüber einer Veränderung von Haar-Kontakt-Eigenschaften, welche beispielsweise mit einer Verwendung von Haarcolorationen, Bleaching, Haarstyling und Haarpflege verbunden sind, sein können.

In verschiedenen Ausführungsbeispielen kann das Signal, beispielsweise eine Welle, ein zeitlicher Verlauf eines Geräuschpegels und/oder ein Frequenzspektrum eines akustischen Signals, mit bestimmten sensorischen Eigenschaften assoziiert oder damit korreliert werden. In verschiedenen Ausführungsbeispielen kann eine Analyse der aufgenommen Signale es ermöglichen, einzelne äußere Haarschädigungsgrade zu charakterisieren und zu unterscheiden, und/oder eine Wirksamkeit von Haarpflegeprodukten darzustellen und zu charakterisieren.

In verschiedenen Ausführungsbeispielen können beispielsweise akustische Signale in einem Frequenzbereich von etwa 86 Hz bis etwa 22011 Hz, z.B. in einem Bereich von etwa 1500 Hz bis etwa 7000 Hz und/oder z.B. von etwa 7000 Hz bis etwa 18000 Hz untersucht werden.

In verschiedenen Ausführungsbeispielen kann ein akustisches Signal, beispielsweise eine (zeitabhängige) Amplitude und/oder eine Frequenz eines akustischen Signals, über einen gesamten Frequenzbereich, beispielsweise einen der oben genannten Frequenzbereiche oder einen Teilbereich davon, integriert beurteilt werden, beispielsweise als ein über den gewählten Frequenzbereich integrierten Pegelwert, und/oder das akustische Signal kann als Frequenzspektrum, entweder für einen gesamten Frequenzbereich, beispielsweise einen der oben genannten Frequenzbereiche oder einen Teilbereich davon, spektral aufgelöst analysiert werden.

Bei einer spektral aufgelösten Analyse kann ein erfasstes Frequenzspektrum in verschiedenen Ausführungsbeispielen mit einer Mehrzahl von Referenz-Frequenzspektren verglichen werden. Dabei kann, beispielsweise auf an sich bekannte Weise, ein ähnlichstes Referenz-Frequenzspektrum ermittelt werden, beispielsweise mittels Ermittelns einer quadratischen Abweichung zwischen dem erfassten Frequenzspektrum und jedem der Referenz-Frequenzspektren, wobei das Referenz-Frequenzspektrum mit der kleinsten quadratischen Abweichung das ähnlichste Frequenzspektrum sein kann.

Die Referenz-Frequenzspektren können in verschiedenen Ausführungsbeispielen empirisch (z.B. im Labor) gewonnene Frequenzspektren für Haare aufweisen, deren Haarschädigungsgrad bekannt sein kann und dem Frequenzspektrum, z.B. mittels einer Datenbank, zugeordnet sein kann. In verschiedenen Ausführungsbeispielen können ferner weitere Informationen über die Haare vorliegen, die als Grundlage für die Referenzspektren dienen, z.B. "viermal gebleichtes Haar - hoher Schädigungsgrad" oder "unbehandeltes Haar - keine Schädigung", und/oder für die Haare, die für das Ermitteln der Referenzspektren genutzt werden, kann eine Entwicklung eines Haarzustands, z.B. des Haarschädigungsgrads, bereitgestellt sein, z.B. mehrere Referenzspektren, von denen jedes nach einer anderen Haarbehandlungsstufe aufgenommen wurde, wobei die Haarbehandlung eine pflegende Haarbehandlung und/oder eine schädigende Haarbehandlung aufweisen kann. Ein Mittel (z.B. Produkt und/oder Inhaltsstoff), welches bei einer Behandlung genutzt wurde, kann außerdem mittels der Datenbank erfasst worden sein.

In verschiedenen Ausführungsbeispielen kann einem Nutzer auch die (z.B. der Datenbank entnommene) Zusatzinformation bereitgestellt werden, beispielsweise welchem Behandlungsgrad sein Haarzustand entspricht, und/oder wie sich sein Haarzustand voraussichtlich entwickeln wird, wenn er eine bestimmte Behandlung vornimmt, z.B. ein bestimmtes Mittel anwendet.

In verschiedenen Ausführungsbeispielen, z.B. bei Verwendung einer Cloud, kann die Datenbank alternativ zu einem Erzeugen im Labor mittels Nutzerdaten erzeugt werden. In verschiedenen Ausführungsbeispielen kann die im Labor erzeugte Datenbank mittels Nutzerdaten, welche mittels der Cloud bereitgestellt werden können, ergänzt werden.

In verschiedenen Ausführungsbeispielen kann ein akustisches Signal, beispielsweise eine (zeitabhängige) Amplitude und/oder eine Frequenz eines akustischen Signals, abhängig sein von einer Geschwindigkeit, mit welcher die Haarinformations-Ermittlungsvorrichtung entlang der Haare des Nutzers bewegt wird.

In verschiedenen Ausführungsbeispielen kann eine Geschwindigkeit, mit welcher die Haarinformations-Ermittlungsvorrichtung entlang der Haare des Nutzers bewegt wird (der Einfachheit halber als Kämmgeschwindigkeit bezeichnet, auch für Fälle, in welchen die Haarinformations-Ermittlungsvorrichtung keine Kammform aufweist) für das Verfahren zum Bereitstellen einer Information bezüglich Haaren eines Nutzers geeignet sein, wenn sie in einem Bereich zwischen mehr als 0 cm/s und etwa 30 cm/s liegt, wobei ein Bereich von 5 cm/s bis etwa 15 cm/s besser geeignet sein kann.

In verschiedenen Ausführungsbeispielen kann die Haarinformations-Ermittlungsvorrichtung eingerichtet sein, die Kämmgeschwindigkeit zu ermitteln, beispielsweise mittels Geschwindigkeitssensoren wie hierin an anderer Stelle beschrieben.

In verschiedenen Ausführungsbeispielen kann die ermittelte Geschwindigkeit genutzt werden, um dem Nutzer eine Rückmeldung bereitzustellen, ob er mit einer für das Verfahren geeigneten Geschwindigkeit kämmt. Beispielsweise kann ein Signalton und/oder ein Signallicht (z.B. rot) und/oder eine Anzeige auf einer gekoppelten Anzeigevorrichtung bereitgestellt werden, wenn das Kämmen mit einer zu geringen oder einer zu hohen Geschwindigkeit erfolgt. In verschiedenen Ausführungsbeispielen kann ferner, beispielsweise mittels eines Signallichts in einer anderen Farbe (z.B. grün) und/oder mittels einer Anzeige auf der Anzeigevorrichtung, der Nutzer darüber informiert werden, dass er mit der richtigen Geschwindigkeit kämmt. In verschiedenen Ausführungsbeispielen kann eine Auswertung des akustischen Signals nur dann erfolgen, wenn das Kämmen mit einer geeigneten Geschwindigkeit erfolgt.

In verschiedenen Ausführungsbeispielen kann ein Auswerten des akustischen Signals bei jeder Kämmgeschwindigkeit (größer als Null) ermöglicht sein.

In verschiedenen Ausführungsbeispielen können mittels der ermittelten Geschwindigkeit aus der Datenbank diejenigen Referenzspektren als Vergleichsspektren oder diejenigen Referenzlautstärken als Vergleichslautstärken ausgewählt werden, welche bei einer vergleichbaren Kämmgeschwindigkeit erzeugt wurden, beispielsweise bei einer Kämmgeschwindigkeit, die um nicht mehr als 20%, z.B. nicht mehr als 10%, von der ermittelten Kämmgeschwindigkeit abweicht.

In verschiedenen Ausführungsbeispielen kann, beispielsweise mittels Laborversuchen, ein Einfluss einer Kämmgeschwindigkeit auf ein Spektrum und/oder eine Lautstärke ermittelt werden und beim Vergleich des erfassten Frequenzspektrums mit dem Referenzspektrum und/oder der Referenzlautstärke berücksichtigt werden. Beispielsweise kann ein Kämmen desselben Haars mit steigender Kämmgeschwindigkeit zu einem Verlagern des akustischen Frequenzspektrums zu höheren Frequenzen und zu einem Erhöhen seiner Amplitude (und damit auch zu einer höheren Lautstärke) führen. Vorab ermittelte Korrekturfaktoren können somit bei jedem der Referenzspektren angewendet werden, welches nicht bei derselben (oder einer ähnlichen) Kämmgeschwindigkeit aufgezeichnet wurde wie das ermittelte Frequenzspektrum (oder umgekehrt kann das Frequenzspektrum an das jeweils zum Vergleich herangezogene Referenzspektrum angepasst werden), so dass ein Vergleichen des ermittelten Frequenzspektrums mit jedem der Referenzspektren der Datenbank ermöglicht wird bzw. ist.

In verschiedenen Ausführungsbeispielen kann eine Lautstärke, welche mittels der Vorrichtung zum Erfassen der akustischen Emission (z.B. des Mikrofons) erfasst werden beim Kämmen der Haare des Nutzers in einem Bereich von etwa -81 dB bis -10 dB, z.B. von etwa -81 dB bis etwa -35 dB liegen. Hierbei entspricht -81 dB einer niedrigsten Lautstärke, welche gerade noch mittels der Vorrichtung zum Erfassen der akustischen Emission (z.B. des Mikrofons) erfassbar sein kann. Da dB eine logarithmische Einheit darstellt, entspricht -35 dB einer Lautstärke, welche um einen Faktor von mehr als 10000 größer ist als die niedrigste Lautstärke, und -10dB entspricht einer Lautstärke, welche beinahe 10⁷ mal lauter ist als die niedrigste Lautstärke, wobei allerdings auch die hohen Lautstärken, verglichen mit Lautstärken von Umgebungsgeräuschen, immer noch vergleichsweise leise sein können.

In verschiedenen Ausführungsbeispielen kann die Haarinformations-Ermittlungsvorrichtung bereitgestellt sein als ein Zubehör, z.B. ein "kluges Zubehör" (auch als "smartes Zubehör" bezeichnet) für ein Smartphone (oder ein ähnliches Gerät wie z.B. ein Tablet, ein iPod o.ä.), welches mit dem Smartphone verbunden, z.B. daran angesteckt werden kann, womit ermöglicht werden kann, Verarbeitungsmöglichkeiten und/oder Sensoren des Smartphones zu nutzen.

In verschiedenen Ausführungsbeispielen kann die Haarinformations-Ermittlungsvorrichtung bereitgestellt sein als eine eigenständige Vorrichtung, welche beispielsweise eine eigene Vorrichtung zum Übertragen von Daten aufweisen kann und somit eine so genannte "Internet-of-Things (loT)"-Vorrichtung sein kann. Die eigenständige Haarinformations-Ermittlungsvorrichtung kann die aufgezeichneten Daten (z.B. akustische Daten und ggf. Geschwindigkeitsdaten) beispielsweise mittels Bluetooth, WiFi, NFC oder ähnlichem an eine externe Datenverarbeitungsvorrichtung übertragen, z.B. zu einer Cloud.

In verschiedenen Ausführungsbeispielen können die aufgezeichneten Daten, wie hierin an anderer Stelle beschrieben, mittels Softwarealgorithmen analysiert werden, beispielsweise mittels Vergleichens von akustischen Frequenzspektren mit bereits aufgenommenen Referenzspektren (oder allgemeiner, von akustischen Daten mit Referenzdaten), um einen Haarschädigungsgrad zu ermitteln.

In verschiedenen Ausführungsbeispielen, beispielsweise wenn die Referenzdaten mittels der Cloud bereitgestellt werden, können diese einem Nutzer jederzeit zur Verfügung stehen, um als Referenzdaten für einen Vergleich genutzt zu werden.

In verschiedenen Ausführungsbeispielen können die mittels der Haarinformations-Ermittlungsvorrichtung erfassten Daten, beispielsweise ein akustisches Frequenzspektrum und/oder eine Lautstärke, und/oder daraus ermittelte Werte und/oder Empfehlungen, gespeichert werden, beispielsweise in einem in der Haarinformations-Ermittlungsvorrichtung integrierten Speicher und/oder in der externen Datenverarbeitungsvorrichtung, z.B. der Cloud. Die gespeicherten Daten können so abgespeichert werden, dass zumindest dem Nutzer ermöglicht ist, diese Daten als seine Daten zu erkennen. Damit kann ein Vergleichen von Haarinformationen, die beispielsweise zu verschiedenen Zeitpunkten gewonnen wurden (z.B. vor und nach einer Behandlung) miteinander ermöglicht werden.

In verschiedenen Ausführungsbeispielen können mittels der Haarinformations-Ermittlungsvorrichtung, ggf. unter Zuhilfenahme einer externen Datenverarbeitungsvorrichtung, Daten von Sensoren wie z.B. einem Mikrofon umgewandelt werden. Dienste einer Cloud können in Anspruch genommen werden für eine präzise Analyse und einen Vergleich der Daten (z.B. einen Vergleich der Daten mit Referenzdaten).

Die Haarinformations-Ermittlungsvorrichtung kann eine Kombination von Sensoren nutzen, beispielsweise mindestens ein Gyroskop und/oder einen Beschleunigungssensor, um eine Position der Haarinformations-Ermittlungsvorrichtung im Raum zu ermitteln. Anhand einer zeitlichen Veränderung der Position im Raum und/oder der Beschleunigung kann außerdem eine Geschwindigkeit der Haarinformations-Ermittlungsvorrichtung ermittelt werden.

In verschiedenen Ausführungsbeispielen, z.B. in einem Fall, dass die Haarinformations-Ermittlungsvorrichtung ein Zubehörteil ist oder aufweist, welches beispielsweise dafür vorgesehen sein kann, an einem Smartphone, z.B. einem iPhone angebracht zu werden, können die (integrierten) Sensoren des Smartphones als die Geschwindigkeitssensoren genutzt werden.

In verschiedenen Ausführungsbeispielen können die Haarinformations-Ermittlungsvorrichtung und/oder das Haarinformations-Ermittlungssystem eine Verbindung zum Übertragen von Daten aufweisen, beispielsweise zwischen einem Smartphone/Tablet, welches Teil des Haarinformations-Ermittlungssystems sein kann, und einer Cloud, und/oder zwischen der Haarinformations-Ermittlungsvorrichtung und einem Smartphone/Tablet, und/oder zwischen einer Haarinformations-Ermittlungsvorrichtung und einer Cloud.

Für die Datenübertragung kann in verschiedenen Ausführungsbeispielen ein bekannter Datenübertragungsstandard genutzt werden, z.B. Bluetooth, WiFi, NFC oder ähnliches. In verschiedenen Ausführungsbeispielen kann die Haarinformations-Ermittlungsvorrichtung bzw. das Haarinformations-Ermittlungssystem eine entsprechende Datenübertragungsvorrichtung zum Senden und/oder Empfangen der Daten aufweisen.

In verschiedenen Ausführungsbeispielen können mittels der Haarinformations-Ermittlungsvorrichtung erhobene Daten (d.h. der ermittelte mindestens eine Sensorwert und/oder basierend darauf ermittelte Daten und/oder Empfehlungen) bereitgestellt werden.

In verschiedenen Ausführungsbeispielen können lediglich die ermittelten Sensorwerte an eine Anzeigevorrichtung (z.B. ein Smartphone oder ähnliches) übermittelt und angezeigt werden.

In verschiedenen Ausführungsbeispielen können die Sensorwerte auf unterschiedliche Weise untersucht (z.B. analysiert und/oder ausgewertet) werden. Dabei kann die Untersuchung sich in verschiedenen Ausführungsbeispielen auf aktuell erhobene Daten (einen aktuellen kosmetischen Zustand (auch als kosmetischer Status bezeichnet)) und/oder auf eine zeitliche Veränderung der Daten (des kosmetischen Status) beziehen.

In verschiedenen Ausführungsbeispielen kann die Analyse durch die Haarinformations-Ermittlungsvorrichtung selbst, beispielsweise mittels der Schaltkreisvorrichtung, erfolgen, und ein Analyseergebnis kann zum Bereitstellen des Analyseergebnisses an eine Anzeigevorrichtung übertragen werden, beispielsweise an ein Display, einen Lautsprecher, ein Smartphone oder ähnliches.

In verschiedenen Ausführungsbeispielen, z.B. vorzugsweise, können die Daten übertragen werden auf/an eine externe Datenverarbeitungsvorrichtung (auch als externe Plattform bezeichnet), z.B. auf ein Smartphone mit App, an eine Cloud, usw. Nach der Datenübertragung auf die externe Datenverarbeitungsvorrichtung kann mittels dieser die Untersuchung der Daten ausgeführt werden, beispielsweise zum Ermitteln einer Empfehlung.

In verschiedenen Ausführungsbeispielen kann die Datenauswertung Grundlage einer Empfehlung, z.B. einer Produkt- und/oder Verfahrensempfehlung sein, z.B. kann die Empfehlung so genannte DOs (anweisende Empfehlungen) und DON`Ts (warnende Empfehlungen) aufweisen, z.B. (als Beispiel für ein DO) "Nimm das Shampoo X und style Deine Haare mit Schaumfestiger Y" oder (als Beispiel für ein DON'T) "Verwende keine basische Dauerwelle".

In verschiedenen Ausführungsbeispielen können dem Konsumenten aktuelle, persönliche Daten hinsichtlich des kosmetischen Zustands seiner Haare, z.B. eines Schädigungsgrads seiner Haaroberfläche (der Haarkutikula) bereitgestellt werden.

In verschiedenen Ausführungsbeispielen kann die Möglichkeit, den kosmetischen (Haarschädigungs-)Zustand seiner Haare selbst zu ermitteln, den Verbraucher von einer Notwendigkeit entbinden, sich einem Fachmann/einer Fachfrau (z.B. Friseur/in) zu bedienen.

In verschiedenen Ausführungsbeispielen kann eine Abstimmung der kosmetischen Behandlung mit individualisierten Konsumentendaten einen iterativen kosmetischen Behandlungszyklus ermöglichen, ein Ergebnis der kosmetischen Behandlung verbessern und/oder eine Motivation des Nutzers, die Behandlung fortzusetzen, erhöhen.

In verschiedenen Ausführungsbeispielen können einem Anwender Informationen über einen Status des Haares (auch als Haarzustand bezeichnet) bereitgestellt werden, welche ferner genutzt werden können, um eine individuelle, auf den Haarzustand des Nutzers abgestimmte Empfehlung zu ermitteln, beispielsweise eine Produktempfehlung (z.B. für ein Haarpflege- und/oder ein Haarstylingprodukt) und/oder eine Pflegeempfehlung, z.B. eine Pflegeempfehlung, welche die Haare des Nutzers betrifft.

In verschiedenen Ausführungsbeispielen kann die Empfehlung direkt mittels der Haarinformations-Ermittlungsvorrichtung ermittelt sein oder werden, d.h. die elektronische Schaltkreisvorrichtung kann eingerichtet sein, die Empfehlung selbst (auch als direkt bezeichnet) zu ermitteln. Beispielsweise kann die elektronische Schaltkreisvorrichtung eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Sensordaten zu empfangen und entweder dem Nutzer direkt bereitzustellen, oder um die Sensordaten zu verwenden, um die Empfehlung bereitzustellen. Beispielsweise können die Sensordaten mit einer Datenbank verglichen werden, welche beispielsweise empirisch gewonnen worden sein kann. In der Datenbank können einer Mehrzahl von Sensordaten, z.B. einer Mehrzahl von Mikrofon-Daten, die z.B. jeweils in Verbindung mit einer Geschwindigkeit stehen können, Empfehlungen zugeordnet sein.

In verschiedenen Ausführungsbeispielen kann die elektronische Schaltkreisvorrichtung eingerichtet sein, die Empfehlung, z.B. Produkt- oder Behandlungsempfehlung, indirekt zu ermitteln. Beispielsweise kann die elektronische Schaltkreisvorrichtung (z.B. zusätzlich zu einem Speicher und einem Prozessor, z.B. einem Mikroprozessor) mit einer Datenübertragungsvorrichtung ausgerüstet sein, welche eingerichtet sein kann, die von der elektronische Schaltkreisvorrichtung empfangenen Sensordaten an eine externe Datenverarbeitungsvorrichtung, z.B. an einen Computer, z.B. eine Cloud, zu übermitteln, mittels derer, beispielsweise wie oben für das Ermitteln der Empfehlung mittels der elektronischen Schaltkreisvorrichtung beschrieben, die Empfehlung ermittelt werden kann, um die Empfehlung bereitzustellen, beispielsweise mittels Übertragens an eine Empfehlung und/oder mittels Übertragens der Empfehlung zurück an die elektronische Schaltkreisvorrichtung (z.B. mittels der Datenübertragungsvorrichtung). In verschiedenen Ausführungsbeispielen kann die Datenübertragung mehrstufig erfolgen, beispielsweise indem die Sensordaten von der Schaltkreisvorrichtung zunächst an die Anzeigevorrichtung (z.B. ein Smartphone, ein Tablet o.ä.) übermittelt werden, und die Anzeigevorrichtung die Sensordaten an die externe Datenverarbeitungsvorrichtung (z.B. die Cloud) überträgt.

In verschiedenen Ausführungsbeispielen kann das Bereitstellen der Empfehlung an den Nutzer ein Bereitstellen mittels eines Übertragens der Empfehlung an eine Anzeigevorrichtung und Anzeigen der Empfehlung aufweisen.

Das Übertragen kann in verschiedenen Ausführungsbeispielen mittels einer drahtlosen Übertragungsvorrichtung erfolgen. Die drahtlose Übertragungsvorrichtung kann beispielsweise Teil der elektronischen Schaltkreisvorrichtung sein. Die drahtlose Übertragungsvorrichtung kann in verschiedenen Ausführungsbeispielen einen Chip oder Tag aufweisen, der die drahtlose Datenübertragung, z.B. mittels Bluetooth, WLAN, ZigBee, NFC, Wibree, Threald, WiMAX oder ähnlichem ermöglicht.

In verschiedenen Ausführungsbeispielen kann die Anzeigevorrichtung einen Computerbildschirm, ein Smartphone, ein Tablet, ein iPad, einen Laptop oder ähnliches aufweisen.

In verschiedenen Ausführungsbeispielen kann ein Haarinformations-Ermittlungssystem gebildet sein von einer Haarinformations-Ermittlungsvorrichtung und der Anzeigevorrichtung.

Als Variation zu den obigen Beispielen können in verschiedenen Ausführungsbeispielen andere Kombinationen von zwei oder mehr Empfehlungen mittels der Anzeigevorrichtung bereitgestellt werden.

In verschiedenen Ausführungsbeispielen kann die Haarinformations-Ermittlungsvorrichtung ferner eine Kamera aufweisen zum Aufnehmen mindestens eines digitalen Bildes von den Haaren des Nutzers. Das von der Kamera aufgenommene Bild kann der elektronischen Schaltkreisvorrichtung bereitgestellt werden. Das Bild kann in verschiedenen Ausführungsbeispielen beispielsweise direkt (z.B. mittels der elektronischen Schaltkreisvorrichtung) oder indirekt (z.B. mittels einer externen Datenverarbeitungsvorrichtung), z.B. mittels Vergleichens mit Referenzdaten, genutzt werden, um eine Haarzustandsinformation zu ermitteln, beispielsweise einen visuellen Schädigungsgrad. Die mittels der Kamera ermittelte Haarzustandsinformation kann einbezogen werden beim Ermitteln einer Empfehlung, z.B. einer Produkt- oder Behandlungsempfehlung.

In verschiedenen Ausführungsbeispielen kann die Kamera ferner genutzt werden bei einem Ermitteln einer Geschwindigkeit, mit welcher der Vorrichtungskörper (in oder an welchem die Kamera angeordnet sein kann) entlang der Haare des Nutzers bewegt wird. Beispielsweise kann mittels der Kamera eine Mehrzahl von Bildern der Haare aufgenommen werden, welche ein Ermitteln eines entlang der Haare zurückgelegten Weges ermöglichen. Unter Einbeziehung von Aufnahmezeitpunkten der digitalen Bilder kann somit die Geschwindigkeit ermittelt werden. Die ermittelte Geschwindigkeit kann, wie hierin an anderer Stelle beschrieben, in Verbindung mit den ermittelten akustischen Signalen ausgewertet werden zum Ermitteln einer Haarzustands- (z.B. Haarschädigungs-)information.

In verschiedenen Ausführungsbeispielen wird eine Haarinformations-Ermittlungsvorrichtung zum Bereitstellen einer Information bezüglich Haaren eines Nutzers bereitgestellt. Die Haarinformations-Ermittlungsvorrichtung kann einen Vorrichtungskörper mit mindestens einem ersten Bereich und einem zweiten Bereich aufweisen, welche so gestaltet sind, dass die Haare des Nutzers zwischen dem ersten Bereich und dem zweiten Bereich bewegbar sind, während sie in Berührung sind mit dem ersten Bereich und dem zweiten Bereich, mindestens ein im oder am Vorrichtungskörper angeordnetes Mikrofon zum Erfassen eines Geräuschs während eines Bewegens der Haare zwischen dem ersten Bereich und dem zweiten Bereich, einen im oder am Vorrichtungskörper angeordneten Geschwindigkeitssensor-Schaltkreis, der mindestens einen Sensor aufweist, zum Ermitteln eines Werts, der eine Geschwindigkeit des Vorrichtungskörpers repräsentiert, und eine im oder am Vorrichtungskörper angeordnete elektronische Schaltkreisvorrichtung, wobei die elektronische Schaltkreisvorrichtung mit dem mindestens einen Mikrofon und dem Geschwindigkeitssensor-Schaltkreis gekoppelt sein kann zum Empfangen des erfassten Geräuschs und der Geschwindigkeit des Vorrichtungskörpers, und wobei die elektronische Schaltkreisvorrichtung eingerichtet sein kann, basierend auf dem empfangenen erfassten Geräusch und der Geschwindigkeit des Vorrichtungskörpers, dem Nutzer eine Information bezüglich seiner Haare bereitzustellen.

In verschiedenen Ausführungsbeispielen kann der Vorrichtungskörper als Kamm oder als Bürste geformt sein, und der erste Bereich und der zweite Bereich können zwei benachbarte Kammzinken oder zwei benachbarte Borsten der Bürste aufweisen.

In verschiedenen Ausführungsbeispielen kann die elektronische Schaltkreisvorrichtung eingerichtet sein, basierend auf dem erfassten Geräusch und der Geschwindigkeit des Vorrichtungskörpers eine Haarzustandsinformation zu ermitteln und dem Nutzer bereitzustellen.

In verschiedenen Ausführungsbeispielen kann die elektronische Schaltkreisvorrichtung eingerichtet sein, basierend auf dem erfassten Geräusch und der Geschwindigkeit des Vorrichtungskörpers eine Empfehlung zu ermitteln und dem Nutzer bereitzustellen.

In verschiedenen Ausführungsbeispielen die Empfehlung mindestens eine aufweisen von einer Haarpflegeproduktempfehlung, einer Haarstylingproduktempfehlung und einer Haarbehandlungsempfehlung.

In verschiedenen Ausführungsbeispielen kann das mindestens eine Mikrofon eine Mehrzahl von Mikrofonen aufweisen.

In verschiedenen Ausführungsbeispielen kann das mindestens eine Mikrofon im Vorrichtungskörper versiegelt angeordnet sein.

In verschiedenen Ausführungsbeispielen kann die elektronische Schaltkreisvorrichtung eine kabellose Datenaustauschvorrichtung aufweisen.

In verschiedenen Ausführungsbeispielen kann die Haarinformations-Ermittlungsvorrichtung ferner eine Kamera zum Aufnehmen eines digitalen Bildes der Haare aufweisen.

In verschiedenen Ausführungsbeispielen wird ein Haarinformations-Ermittlungssystem bereitgestellt. Das Haarinformations-Ermittlungssystem kann eine Haarinformations-Ermittlungsvorrichtung gemäß verschiedenen Ausführungsbeispielen aufweisen, und eine Anzeigevorrichtung, wobei die mindestens eine Haarinformations-Ermittlungsvorrichtung eingerichtet sein kann, der Anzeigevorrichtung die Information bezüglich der Haare des Nutzers mittels der Datenaustauschvorrichtung zu übermitteln.

In verschiedenen Ausführungsbeispielen kann die Anzeigevorrichtung einen Computerbildschirm, ein Smartphone, ein Tablet, ein iPad oder einen Laptop aufweisen.

In verschiedenen Ausführungsbeispielen wird ein Haarinformations-Ermittlungssystem zum Bereitstellen einer Information bezüglich Haaren eines Nutzers bereitgestellt.

Das Haarinformations-Ermittlungssystem kann einen Vorrichtungskörper mit mindestens einem ersten Bereich und einem zweiten Bereich aufweisen, welche so gestaltet sind, dass die Haare des Nutzers zwischen dem ersten Bereich und dem zweiten Bereich bewegbar sind, während sie in Berührung sind mit dem ersten Bereich und dem zweiten Bereich, mindestens ein im oder am Vorrichtungskörper angeordnetes Mikrofon zum Erfassen eines Geräuschs während eines Bewegens der Haare zwischen dem ersten Bereich und dem zweiten Bereich, eine im oder am Vorrichtungskörper angeordnete elektronische Schaltkreisvorrichtung, eine Datenverarbeitungsvorrichtung mit einem Gehäuse, einen in dem Gehäuse der Datenverarbeitungsvorrichtung angeordneten Geschwindigkeitssensor-Schaltkreis, der mindestens einen Sensor aufweist, zum Ermitteln eines Werts, der eine Geschwindigkeit der Datenverarbeitungsvorrichtung repräsentiert, wobei der Vorrichtungskörper eingerichtet ist, mit der Datenverarbeitungsvorrichtung fest verbunden zu werden, so dass die Geschwindigkeit der Datenverarbeitungsvorrichtung gleich ist der Geschwindigkeit des Vorrichtungskörpers, wobei die elektronische Schaltkreisvorrichtung mit dem mindestens einen Mikrofon gekoppelt ist zum Empfangen des erfassten Geräuschs, wobei die Datenverarbeitungsvorrichtung mit dem Geschwindigkeitssensor-Schaltkreis gekoppelt ist zum Empfangen der Geschwindigkeit des Vorrichtungskörpers, und wobei die Datenverarbeitungsvorrichtung eingerichtet ist, basierend auf dem empfangenen erfassten Geräusch und der Geschwindigkeit des Vorrichtungskörpers, dem Nutzer eine Information bezüglich seiner Haare bereitzustellen.

In verschiedenen Ausführungsbeispielen kann die Datenverarbeitungsvorrichtung ein Smartphone oder ein Tablet aufweisen.

In verschiedenen Ausführungsbeispielen wird ein Verfahren zum Bereitstellen einer Information bezüglich Haaren eines Nutzers bereitgestellt. Das Verfahren kann aufweisen ein
Bewegen einer Haarinformations-Ermittlungsvorrichtung gemäß verschiedenen Ausführungsbeispielen derart, dass sich die Haare des Nutzers zwischen dem ersten Bereich und dem zweiten Bereich bewegen, während sie in Berührung sind mit dem ersten Bereich und dem zweiten Bereich, ein Erfassen eines Geräuschs mittels des mindestens einen Mikrofons und einer Geschwindigkeit des Vorrichtungskörpers mittels des Geschwindigkeitssensor-Schaltkreises während des Bewegens des Vorrichtungskörpers, und ein Bereitstellen einer Information bezüglich der Haare des Nutzers, basierend auf dem erfassten Geräusch und der Geschwindigkeit des Vorrichtungskörpers.

In verschiedenen Ausführungsbeispielen kann das Verfahren ferner ein Ermitteln mindestens einer Haarzustandsinformation basierend auf dem erfassten Geräusch und der Geschwindigkeit des Vorrichtungskörpers aufweisen.

In verschiedenen Ausführungsbeispielen kann das Verfahren ferner ein Ermitteln mindestens einer Empfehlung basierend auf dem erfassten Geräusch und der Geschwindigkeit des Vorrichtungskörpers aufweisen.

In verschiedenen Ausführungsbeispielen kann die Empfehlung mindestens eine aufweisen von einer Haarpflegeproduktempfehlung, einer Haarstylingproduktempfehlung und einer Haarbehandlungsempfehlung.

In verschiedenen Ausführungsbeispielen kann das Verfahren ferner ein Übermitteln des erfassten Geräuschs und der erfassten Geschwindigkeit an eine externe Datenverarbeitungsvorrichtung aufweisen, und ein Empfangen der von der externen Datenverarbeitungsvorrichtung bereitgestellten Information, wobei das Ermitteln der mindestens einen Haarzustandsinformation basierend auf dem erfassten Geräusch und der Geschwindigkeit mittels der externen Datenverarbeitungsvorrichtung erfolgen kann.

In verschiedenen Ausführungsbeispielen kann die externe Datenverarbeitungsvorrichtung eine Cloud aufweisen oder sein.

Ausführungsbeispiele der Erfindung sind in den Figuren dargestellt und werden im Folgenden näher erläutert.

Es zeigen
Figur 1 eine schematische Darstellung einer Haarinformations-Ermittlungsvorrichtung gemäß verschiedenen Ausführungsbeispielen;
Figur 2A eine schematische Darstellung eines Haarinformations-Ermittlungssystems gemäß verschiedenen Ausführungsbeispielen in Verbindung mit einer externen Datenverarbeitungsvorrichtung;
Figur 2B eine schematische Darstellung eines Haarinformations-Ermittlungssystems gemäß verschiedenen Ausführungsbeispielen;
Figur 3A eine schematische Darstellung einer Anwendung eines Haarinformations-Ermittlungssystems gemäß verschiedenen Ausführungsbeispielen;
Figur 3B eine schematische Darstellung einer Anwendung eines Haarinformations-Ermittlungssystems gemäß verschiedenen Ausführungsbeispielen;
Figur 4A und Figur 4B zeigen beispielhafte Messwerte eines Mikrofons einer Haarinformations-Ermittlungsvorrichtung gemäß verschiedenen Ausführungsbeispielen; und
Figur 5 ein Flussdiagramm eines Verfahrens zum Bereitstellen einer Haarzustandsinformation gemäß verschiedenen Ausführungsbeispielen.

In der folgenden ausführlichen Beschreibung wird auf die beigefügten Zeichnungen Bezug genommen, die Teil der vorliegenden Anmeldung bilden und in denen zur Veranschaulichung spezifische Ausführungsformen gezeigt sind, in denen die Erfindung ausgeübt werden kann. In dieser Hinsicht wird Richtungsterminologie wie etwa "oben", "unten", "vorne", "hinten", "vorderes", "hinteres", usw. mit Bezug auf die Orientierung der beschriebenen Figur(en) verwendet. Da Komponenten von Ausführungsformen in einer Anzahl verschiedener Orientierungen positioniert werden können, dient die Richtungsterminologie zur Veranschaulichung und ist auf keinerlei Weise einschränkend. Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen verschiedenen beispielhaften Ausführungsformen miteinander kombiniert werden können, sofern nicht spezifisch anders angegeben. Die folgende ausführliche Beschreibung ist deshalb nicht in einschränkendem Sinne aufzufassen, und der Schutzumfang der vorliegenden Erfindung wird durch die angefügten Ansprüche definiert.

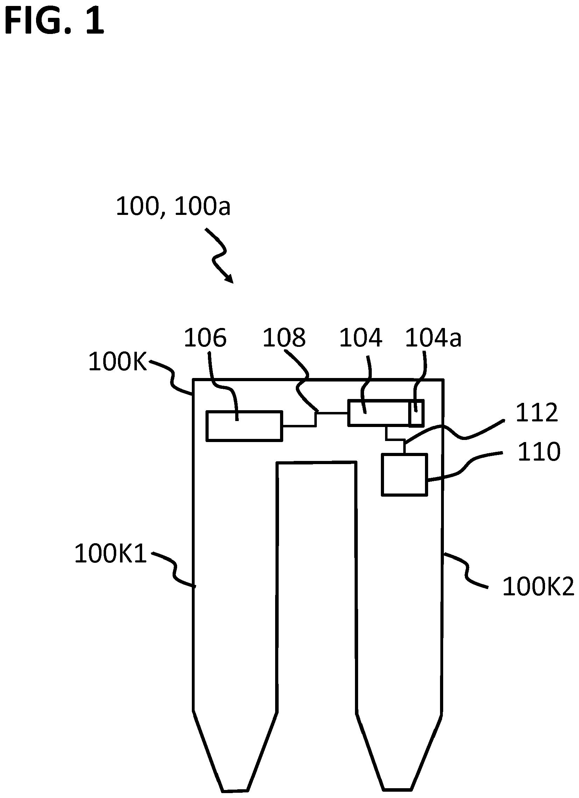

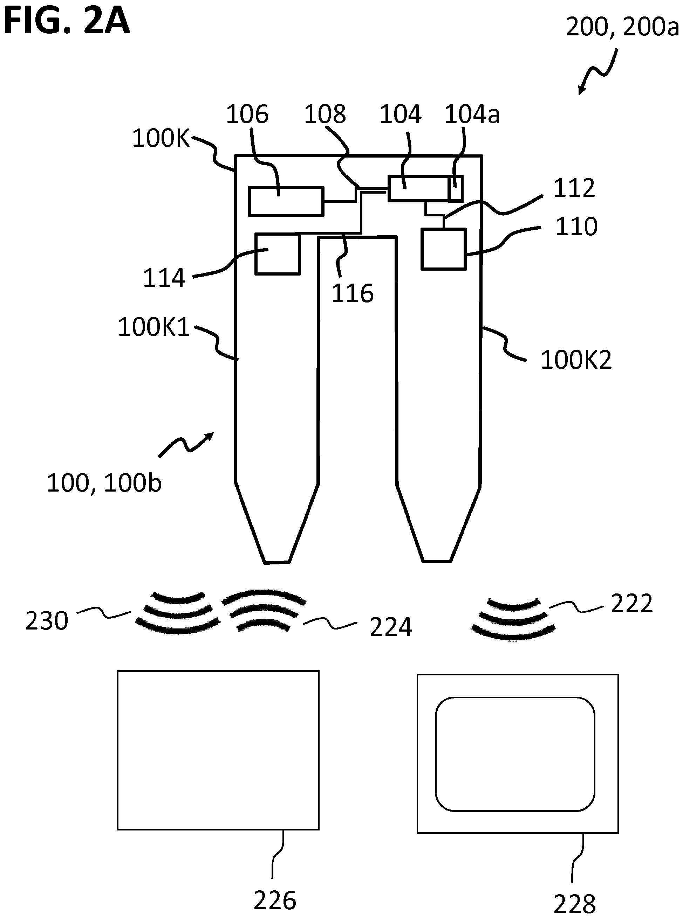

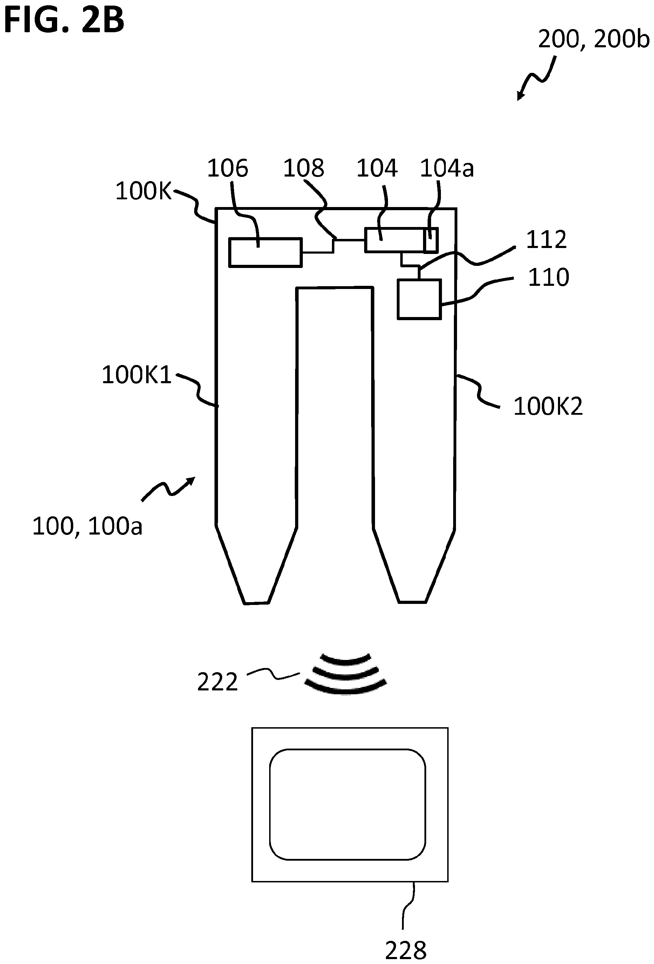

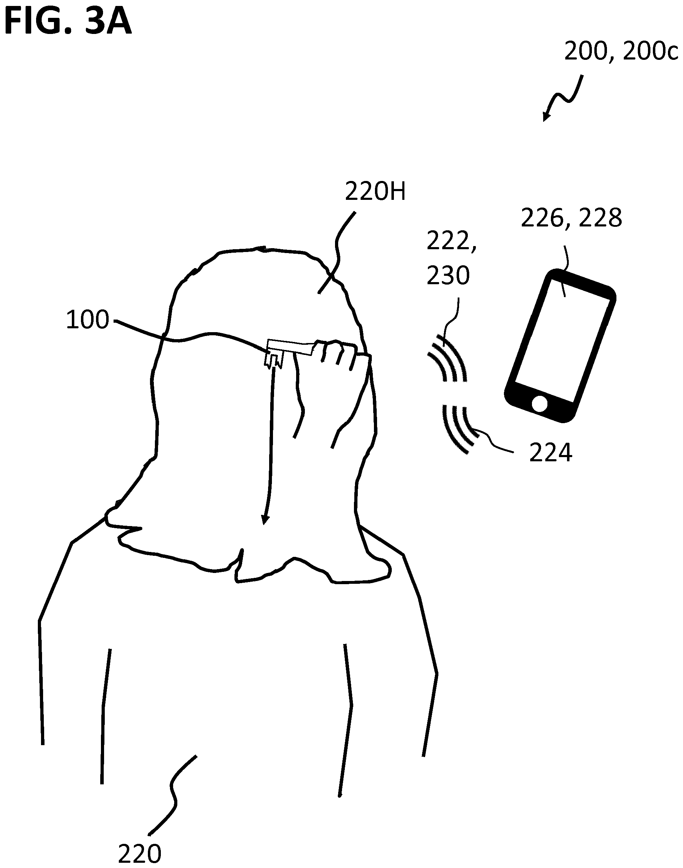

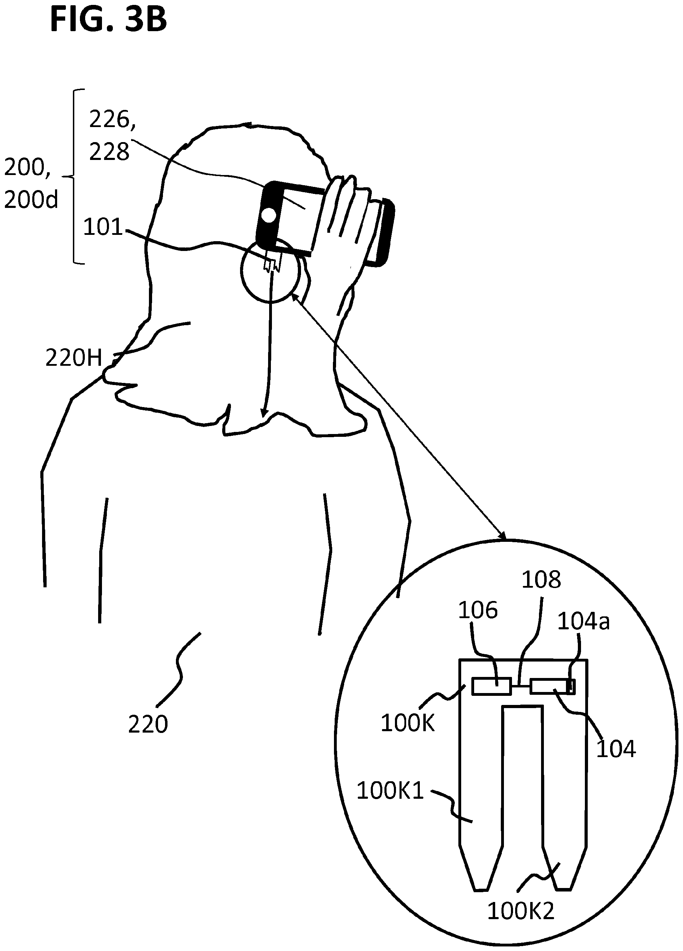

FIG. 1 zeigt eine schematische Darstellung einer Haarinformations-Ermittlungsvorrichtung 100, 100a gemäß verschiedenen Ausführungsbeispielen, FIG. 2A zeigt eine schematische Darstellung eines Haarinformations-Ermittlungssystems 200, 200a gemäß verschiedenen Ausführungsbeispielen in Verbindung mit einer externen Datenverarbeitungsvorrichtung, FIG. 2B zeigt eine schematische Darstellung eines Haarinformations-Ermittlungssystems 200b gemäß verschiedenen Ausführungsbeispielen, FIG. 3A zeigt eine schematische Darstellung einer Anwendung eines Haarinformations-Ermittlungssystems 200, 200c gemäß verschiedenen Ausführungsbeispielen, und FIG. 3B eine schematische Darstellung einer Anwendung eines Haarinformations-Ermittlungssystems 200, 200d gemäß verschiedenen Ausführungsbeispielen.

In verschiedenen Ausführungsbeispielen wird ein Haarinformations-Ermittlungsvorrichtung 100 (verschiedene Ausführungsbeispiele sind als 100a und 100b gekennzeichnet) bereitgestellt.

In verschiedenen Ausführungsbeispielen kann die Haarinformations-Ermittlungsvorrichtung 100 einen Vorrichtungskörper 100K aufweisen.

Der Vorrichtungskörper 100K kann aus einem festen Material, z.B. Kunststoff oder Metall, gebildet sein oder ein solches Material aufweisen. Beispielsweise kann der Vorrichtungskörper 100K aus einem Material gebildet sein oder ein Material aufweisen, welches üblicherweise für einen Kamm, eine Bürste oder ein Glätteisen verwendet wird.

Der Vorrichtungskörper 100K kann in verschiedenen Ausführungsbeispielen mindestens einem ersten Bereich 100K1 und einem zweiten Bereich 100K2 aufweisen, welche so gestaltet sein können, dass Haare 220H eines Nutzers zwischen dem ersten Bereich 100K1 und dem zweiten Bereich 100K2 bewegbar sind, während sie in Berührung sind mit dem ersten Bereich 100K1 und dem zweiten Bereich 100K2, beispielsweise so, dass die Haare an dem ersten Bereich 100K1 und dem zweiten Bereich 100K2 entlanggleiten.

Die Haarinformations-Ermittlungsvorrichtung 100 (oder zumindest der Vorrichtungskörper 100K) kann gemäß verschiedenen Ausführungsbeispielen dafür vorgesehen sein, in einer Kämmbewegung am Haar 220H des Nutzers 220 entlanggeführt zu werden, und zwar auf eine solche Weise, dass während des Ausführens der Kämmbewegung die Haare 220H des Nutzers 220 sich zwischen dem ersten Bereich 100K1 und dem zweiten Bereich 100K2 entlangbewegen, wobei sie in Kontakt sind mit dem ersten Bereich 100K1 und dem zweiten Bereich 100K2.

Hierbei ist darunter, dass die Haare 220H in Kontakt sind mit dem ersten Bereich 100K1 und dem zweiten Bereich 100K2, zu verstehen, dass sich eine Mehrzahl von Haaren 220H, beispielsweise eine Strähne oder ein Büschel, zwischen dem ersten Bereich 100K1 und dem zweiten Bereich 100K2 befindet, ein Teil der Mehrzahl von Haaren 220H in Kontakt ist mit dem ersten Bereich 100K1 und ein Teil der Mehrzahl von Haaren 220H in Kontakt ist mit dem zweiten Bereich 100K2. In verschiedenen Ausführungsbeispielen können eines, mehrere oder alle Haare 220H der Mehrzahl von Haaren 220H in Kontakt sein mit sowohl dem ersten Bereich 100K1 und dem zweiten Bereich 100K2.

In verschiedenen Ausführungsbeispielen kann die Haarinformations-Ermittlungsvorrichtung 100 als Kamm oder kammähnliche Vorrichtung gestaltet sein. Dann können der erste Bereich 100K1 und der zweite Bereich 100K2 beispielsweise als (benachbarte) Kammzinken gebildet sein. In verschiedenen Ausführungsbeispielen kann bei einem Kamm mit mehr als zwei Zinken jeder Zwischenraum zwischen benachbarten Zinken als ein Zwischenraum zwischen einem ersten Bereich 100K1 und einem zweiten Bereich 100K2 betrachtet werden, d.h. der Kamm kann so verstanden werden, dass er eine Mehrzahl von benachbarten ersten und zweiten Bereichen aufweist, zwischen welchen jeweils ein Zwischenraum angeordnet ist, in welchem die Haare 220H des Nutzers 220 so zu bewegen sind, dass sie an den beiden Kammzinken entlanggleiten. Dies kann gegeben sein bei einem herkömmlichen Kämmvorgang.

Ein solcher Kämmvorgang ist in FIG. 3A und FIG. 3B schematisch dargestellt. Um zu veranschaulichen, dass sich während eines (als Pfeil angedeuteten) Bewegens der Haarinformations-Ermittlungsvorrichtung 100 entlang der Haare 220H des Nutzers 220 ein Teil der Haare 220H zwischen den Kammzinken, welche den ersten Bereich 100K1 und den zweiten Bereich 100K2 bilden, befindet, ist die Haarinformations-Ermittlungsvorrichtung 100 in FIG. 3A und FIG. 3B so dargestellt, dass im Wesentlichen nur ein Verbindungsstück zu sehen ist, welches den ersten Bereich 100K1 und den zweiten Bereich 100K2 verbindet.

In verschiedenen Ausführungsbeispielen kann es (für ein Vergleichen mit Referenzdaten, wie hierin an anderer Stelle beschrieben) nötig sein, die Haare 220H des Nutzers 220 von den Haarwurzeln in Richtung der Haarspitzen zu kämmen.

Hierin kann ein Vorgang, die Haare 220H zwischen dem ersten Bereich 100K1 und dem zweiten Bereich 100K2 zu bewegen, als "kämmen" bezeichnet werden, auch dann, wenn die Haarinformations-Ermittlungsvorrichtung 100 nicht als Kamm gestaltet ist.

In verschiedenen Ausführungsbeispielen kann die Haarinformations-Ermittlungsvorrichtung 100 beispielsweise als Bürste oder mit einer bürstenähnlichen Gestaltung, beispielsweise mit nicht, wie bei einem Kamm, entlang zur einer Richtung angeordneter Zinken, sondern mit entlang zwei Richtungen angeordneten Borsten, gebildet sein, oder als ein Glätteisen oder mit einer glätteisenähnlichen Gestaltung, d.h. mit einander gegenüberliegenden Flächen, welche gestaltet sind, die Haare 220H zwischen sich flach zu drücken.

In verschiedenen Ausführungsbeispielen kann die Haarinformations-Ermittlungsvorrichtung 100 mindestens einen im oder am Vorrichtungskörper angeordneten Sensor 106 zum Erfassen akustischer Emissionen aufweisen, beispielsweise ein Mikrofon 106 und/oder einen Beschleunigungssensor 106 (der geeignet sein kann, Beschleunigungen infolge akustischer Emissionen in einem gewissen Frequenzbereich zu erfassen) oder ähnliches. Wie oben beschrieben, kann der Sensor zum Erfassen akustischer Emissionen 106 vereinfachend als Mikrofon 106 bezeichnet werden.

In verschiedenen Ausführungsbeispielen kann das Mikrofon 106 gestaltet sein zum Erfassen eines Geräuschs während des Bewegens der Haare 220H zwischen dem ersten Bereich 100K1 und dem zweiten Bereich 100K2, beispielsweise während des Kämmvorgangs. In verschiedenen Ausführungsbeispielen kann das Mikrofon 106 so in oder an dem Vorrichtungskörper angeordnet sein, dass es geeignet ist zum Erfassen von Geräuschen, die erzeugt werden können beim Kämmen durch ein Gleiten der Haare 220H an dem ersten Bereich 100K1 und/oder dem zweiten Bereich 100K2 und ggf. durch ein Gleiten der Haare 220H aneinander.

In verschiedenen Ausführungsbeispielen kann das Mikrofon 106 so im Vorrichtungskörper 100K angeordnet sein, dass eine Übertragung der Kämmgeräusche zum mindestens einen Mikrofon 106 ermöglicht, z.B. optimiert ist. Das mindestens eine Mikrofon 106 kann beispielsweise in mindestens einem der Kammzinken 100K1 oder 100K2 angeordnet sein.

In verschiedenen Ausführungsbeispielen kann das Mikrofon 106 im Vorrichtungskörper 100K eingebaut sein, beispielsweise versiegelt eingebaut sein.

Damit kann ermöglicht sein, dass die H-E unempfindlich ist gegen Feuchtigkeit und Schmutz. Beispielsweise kann mittels des Versiegelns erreicht werden, dass die H-E gereinigt werden kann, ohne das Mikrofon 106 oder eine andere Vorrichtung zu beschädigen. Das Mikrofon 106 kann beispielsweise bei einem Spritzgießen des Vorrichtungskörpers 100K mit eingegossen werden.

In verschiedenen Ausführungsbeispielen kann die Haarinformations-Ermittlungsvorrichtung 100 einen im oder am Vorrichtungskörper 100K angeordneten Geschwindigkeitssensor-Schaltkreis 110 aufweisen, der mindestens einen Sensor aufweisen kann. Der Geschwindigkeitssensor-Schaltkreis kann gestaltet sein zum Ermitteln eines Werts, der eine Geschwindigkeit des Vorrichtungskörpers 100K repräsentiert. Der Geschwindigkeitssensor-Schaltkreis 110 kann in verschiedenen Ausführungsbeispielen wie oben beschrieben ausgeführt sein. Beispielsweise kann der mindestens eine Sensor zum Ermitteln der Geschwindigkeit einen Beschleunigungssensor und/oder ein Gyroskop und/oder eine Kamera, und/oder jede andere herkömmliche geeignete Vorrichtung, welche zum Ermitteln der Geschwindigkeit des Vorrichtungskörpers geeignet ist, aufweisen. Zum Ermitteln der Geschwindigkeit kann zusätzlich zum jeweiligen Sensorsignal eine Zeitinformation berücksichtigen. Der Wert, der eine Geschwindigkeit des Vorrichtungskörpers 100K repräsentiert, kann ein Geschwindigkeitswert sein, ein zur Geschwindigkeit proportionaler Wert, eine Geschwindigkeitsklassifizierung (z.B. geeignet/ungeeignet oder zu niedrig/geeignet/zu hoch, oder eine Klassifizierung mit mehr als drei Klassen), oder jeder andere die Geschwindigkeit repräsentierende Wert, der ein Auswählen geeigneter Referenzwerten/-spektren für die gemessenen Mikrofondaten erlaubt, beispielsweise ein Auswählen aus in einer Datenbank hinterlegten Referenzwerten/-spektren.

In verschiedenen Ausführungsbeispielen kann die H-E eine im oder am Vorrichtungskörper 100K angeordnete elektronische Schaltkreisvorrichtung 104 aufweisen.

Die elektronische Schaltkreisvorrichtung 104 kann in verschiedenen Ausführungsbeispielen mit dem mindestens einen Mikrofon 106 gekoppelt sein, beispielsweise mittels einer Verbindung 108, zum Empfangen des erfassten Geräuschs. Beim Vorhandensein einer Mehrzahl von Mikrofonen 106 kann die Schaltkreisvorrichtung 104 eine eigene Kopplung zu jedem der Mikrofone 106 aufweisen. Die Kopplung kann in verschiedenen Ausführungsbeispielen eine elektrisch leitende Verbindung, eine (Glas-)faserverbindung und/oder eine kabellose Verbindung aufweisen oder sein. Die elektronische Schaltkreisvorrichtung 104 kann zum Empfangen des mindestens einen Sensorwerts von dem mindestens einen Sensor 106 eingerichtet sein.

Mittels einer Nutzung einer Mehrzahl von Mikrofonen kann ein Signal-zu-Rauschen-Verhältnis verbessert werden.

Die elektronische Schaltkreisvorrichtung 104 kann in verschiedenen Ausführungsbeispielen mit dem Geschwindigkeitssensor-Schaltkreis 110 gekoppelt sein, beispielsweise mittels einer Verbindung 112, zum Empfangen der Geschwindigkeit des Vorrichtungskörpers 100K.

In verschiedenen Ausführungsbeispielen kann die elektronische Schaltkreisvorrichtung eingerichtet sein, basierend auf dem empfangenen erfassten Geräusch und der Geschwindigkeit des Vorrichtungskörpers 100K, dem Nutzer 220 eine Information bezüglich seiner Haare 220H bereitzustellen.

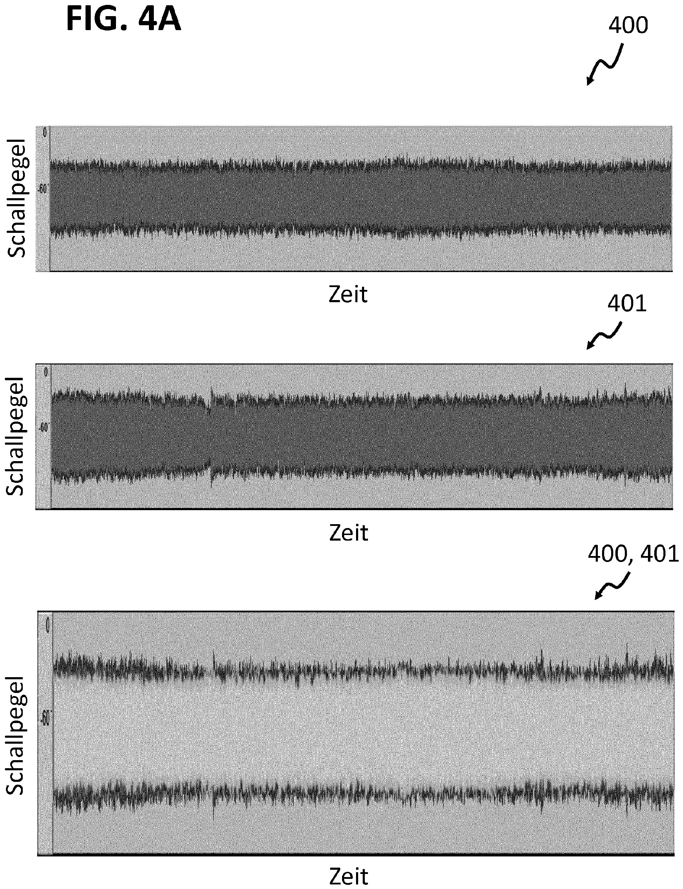

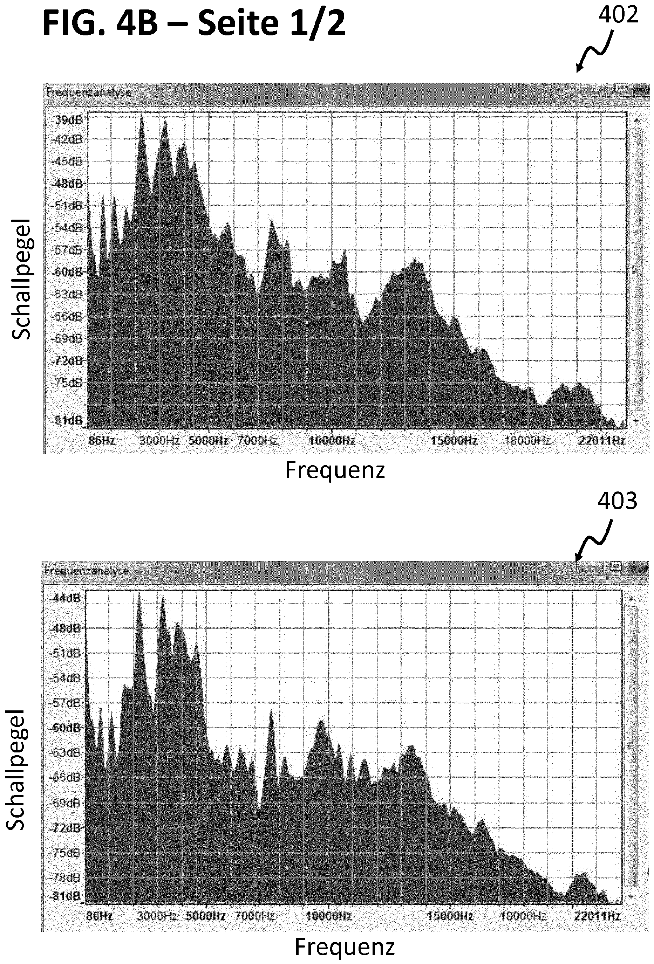

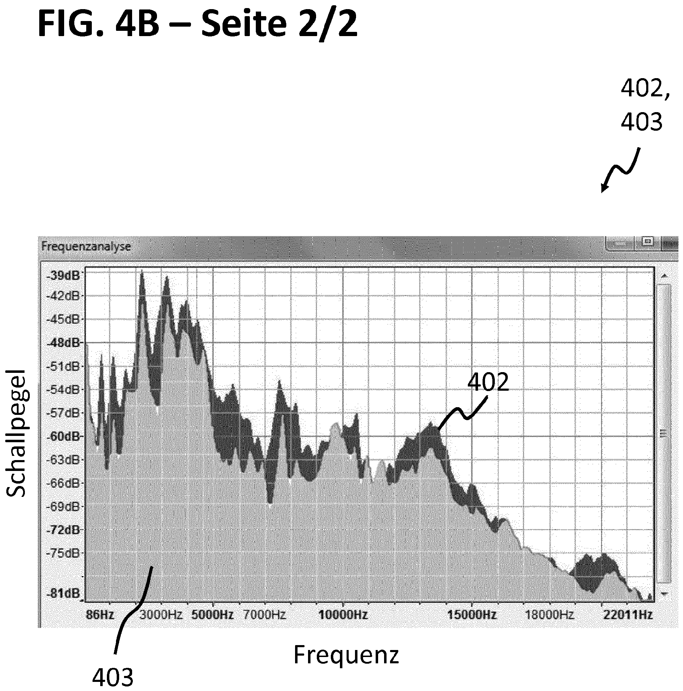

Ferner kann die elektronische Schaltkreisvorrichtung 104 in verschiedenen Ausführungsbeispielen eingerichtet sein, den empfangenen mindestens einen Sensorwert dem Nutzer 220 bereitzustellen, beispielsweise als im Wesentlichen unbearbeitetes Signal, z.B. als zeitlich veränderlichen Schallpegel (siehe dazu als Beispiel FIG. 4A), und/oder als bearbeitetes Signal, z.B. ein akustisches Frequenzspektrum (siehe dazu als Beispiel FIG. 4B).

In verschiedenen Ausführungsbeispielen kann die elektronische Schaltkreisvorrichtung 104 eine Datenverarbeitungsvorrichtung sein oder aufweisen, z.B. kann sie mit einem Speicher und einem Prozessor, z.B. einem Mikroprozessor, ausgerüstet sein, welche eingerichtet sein können, z.B. mittels einer Programmierung, die Daten vom Mikrofon 106 zu empfangen und entweder dem Nutzer 220 direkt bereitzustellen, oder um die Mikrofondaten zu verwenden, um die Empfehlung bereitzustellen.

Die elektronische Schaltkreisvorrichtung 104 kann in verschiedenen Ausführungsbeispielen eingerichtet sein, basierend auf den empfangenen Daten vom Mikrofon 102 mindestens eine Empfehlung zu ermitteln und dem Nutzer 220 bereitzustellen.

In verschiedenen Ausführungsbeispielen können der Geschwindigkeitssensor-Schaltkreis 110 und die Schaltkreisvorrichtung 104 eine integrierte Schaltkreisvorrichtung bilden (nicht dargestellt).

In verschiedenen Ausführungsbeispielen kann die H-E, wie beispielsweise in FIG. 2A anhand der H-E 100c dargestellt, ferner eine Kamera 114 aufweisen. Die Kamera 114 kann mittels einer Verbindung 116 mit der elektronischen Schaltkreisvorrichtung 104 gekoppelt sein. Die Kamera 114 kann, wie oben beschrieben, genutzt werden für ein Ermitteln des Schädigungsgrads des Haares, und/oder für ein Ermitteln einer Geschwindigkeit. In einem Fall, dass die Kamera 114 zumindest unter anderem auch für das Ermitteln der Geschwindigkeit genutzt wird, kann die Kamera 114 in verschiedenen Ausführungsbeispielen ferner mittels einer Verbindung (nicht dargestellt) mit dem Geschwindigkeitssensor-Schaltkreis 110 verbunden sein.

Wie in den schematischen Darstellungen einer Anwendung von Haarinformations-Ermittlungssystemen 200, 200c, 200d gemäß verschiedenen Ausführungsbeispielen dargestellt ist, können Kopfhaare 220H eines Nutzers 220 mittels der H-E gekämmt werden (d.h. die Haare 220H können zwischen dem ersten Bereich 100K1 und dem zweiten Bereich 100K2 bewegt werden, während sie in Kontakt sind mit dem ersten Bereich 100K1 und dem zweiten Bereich 100K2).

Durch das Kämmen erzeugte akustische Signale können mittels des mindestens einen Mikrofons 106 erfasst und bereitgestellt werden.

FIG. 4A und FIG. 4B zeigen beispielhafte Messwerte des Mikrofons 106 einer Haarinformations-Ermittlungsvorrichtung 100, 101 gemäß verschiedenen Ausführungsbeispielen.

In FIG. 4A ist in der oberen Darstellung 400 ein vom Mikrofon 106 gemessener zeitabhängiger Schallpegel dargestellt, welcher bei einem Kämmen von Haar mit äußerst geringer äußerer Haarschädigung erfasst wurde, und in der mittleren Darstellung 401 ist ein zeitabhängiger Schallpegel dargestellt, welcher bei einem Kämmen sehr stark geschädigten Haars erfasst wurde.

Wie besonders anhand einer Überlagerung der beiden Signale (in der unteren Darstellung von FIG. 4A abgebildet) ersichtlich ist, weist der Schallpegel des Kämmens des geschädigten Haars eine höhere Amplitude, und damit eine höhere Lautstärke, auf.

Eine solche Untersuchung der Lautstärke des Signals, beispielsweise für vorgegebene Frequenzbereiche, kann genutzt werden, um einen (äußeren) Haarschädigungsgrad der Haare 220H des Nutzers 220 zu ermitteln.

Die folgende Tabelle stellt einen beispielhaften Datenbankeintrag dar, welcher genutzt werden kann, um eine ermittelte Lautstärke mit in der Datenbank bereitgestellten Lautstärken zu vergleichen. Dabei kann mittels einer mittels des Geschwindigkeitssensor-Schaltkreises 110 ermittelten Kämmgeschwindigkeit sichergestellt sein, dass bei einem Ermitteln der Referenzlautstärken eine vergleichbare Geschwindigkeit genutzt wurde.

| Frequenzbereich | Lautstärke | Haarschädigungsgrad |
|---|---|---|
| 1500 - 7000 Hz | > -43 dB | sehr gering |
| 1500 - 7000 Hz | > -42 dB | gering |
| 1500 - 7000 Hz | > -41 dB | mittel |
| 1500 - 7000 Hz | > -40 dB | stark |
| 1500 - 7000 Hz | > -39 dB | sehr stark |

In FIG. 4B sind Frequenzspektren der erfassten Geräusche aus FIG. 4A dargestellt (Darstellung 402: sehr geringe äußere Schädigung, Darstellung 403: sehr starke äußere Schädigung, Seite 2: Überlagerung von 402 und 403).

Wie insbesondere anhand der Überlagerung ersichtlich ist, führt bei fast jeder Frequenz das geschädigte Haar 220H beim Kämmen zu lauteren akustischen Emissionen.

In verschiedenen Ausführungsbeispielen kann zum Ermitteln eines am besten mit dem erfassten Spektrum übereinstimmenden Referenzspektrums der gesamte Spektralbereich betrachtet werden.

In verschiedenen Ausführungsbeispielen können mittels einer Untersuchung der Referenzspektren besonders geeignete Frequenzbereiche identifiziert werden, welche eine leichte Unterscheidung der Schädigungsgrade ermöglichen, beispielsweise Bereiche, in welchen sich die Frequenzspektren von wenig geschädigtem Haar und stark geschädigtem Haar stark unterscheiden, im Beispiel aus FIG. 4B z.B. ein Bereich zwischen etwa 5000 Hz und etwa 9000 Hz.

Den Referenzspektren, die zum Vergleich herangezogen werden, können, ähnlich der beispielhaften Tabelle für die Lautstärken, Schädigungsgrade zugeordnet sein. Dem Haar des Nutzers 220 kann somit der Schädigungsgrad zugeordnet werden, der dem ähnlichsten Referenzspektrum entspricht.

Mittels der Frequenzanalyse können demnach in verschiedenen Ausführungsbeispielen Aussagen über den äußeren Haarschädigungsgrad getroffen werden.

In verschiedenen Ausführungsbeispielen kann eine Datenbank ferner eine Produktempfehlung auf Basis des Haarschädigungsgrads bereitstellen.

Wie in der folgenden Tabelle dargestellt kann dem Haarschädigungsgrad in der Datenbank beispielsweise mindestens eine Produktempfehlung (oder auch eine Styling- oder sonstige Haarbehandlungsempfehlung) zugeordnet sein.

| Haarschädigungsgrad | Produktempfehlung |
|---|---|
| sehr gering | Produkt mit sehr geringem Pfleganteil |
| gering | Produkt mit geringem Pflegeanteil |
| mittel | Produkt mit mittlerem Pflegeanteil |
| stark | Produkt mit hohem Pflegeanteil |
| sehr stark | Produkt mit sehr hohem Pflegeanteil |

Die erfassten (gemessenen) Sensorwerte können, wie oben beschrieben, entweder direkt in der Haarinformations-Ermittlungsvorrichtung 100 ausgewertet werden, z.B. mittels der elektronischen Schaltkreisvorrichtung 104, oder indirekt ausgewertet werden, indem sie an eine externe Datenverarbeitungsvorrichtung 226 übertragen werden, um dort ausgewertet zu werden, z.B. wie oben beschrieben. Dabei können die Sensordaten oder Teile der Sensordaten in verschiedenen Ausführungsbeispielen ausgewertet werden mittels eines Vergleichs mit (z.B. empirisch gewonnenen) Datenbankeinträgen.

In verschiedenen Ausführungsbeispielen kann ein ermittelter Haarschädigungsgrad einbezogen werden bei einem Ermitteln einer Empfehlung, z.B. einer Produkt- oder Behandlungsempfehlung.

In verschiedenen Ausführungsbeispielen kann die Haarinformations-Ermittlungsvorrichtung 100 in Verbindung mit einer Anzeigevorrichtung 228 und/oder einer externen Datenverarbeitungsvorrichtung 226 als ein Haarinformations-Ermittlungssystem 200 betrachtet werden. In FIG. 2A und FIG. 2B sind beispielhafte Haarinformations-Ermittlungssysteme 200 dargestellt.

Wie oben beschrieben kann die elektronische Schaltkreisvorrichtung 104 in verschiedenen Ausführungsbeispielen eingerichtet sein, basierend auf dem empfangenen mindestens einen Sensorwert (dem mittels des Mikrofons erfassten Geräusch und der Geschwindigkeit) mindestens eine Information und/oder eine Empfehlung zu ermitteln und einem Nutzer bereitzustellen, indem sie wie z.B. in FIG. 2B dargestellt, die Information und/oder Empfehlung selbst (direkt) ermittelt, beispielsweise wie oben beschrieben, z.B. mittels Vergleichens mit in einem Speicher der elektronischen Schaltkreisvorrichtung 104 gespeicherten Referenzspektren, -werten, - wertebereichen und/oder Daten in Datenbanken, wobei den Referenzwerten, -wertebereichen bzw. Daten in Datenbanken jeweils mindestens eine Empfehlung zugeordnet sein kann.

Wie oben beschrieben kann die elektronische Schaltkreisvorrichtung 104 in verschiedenen Ausführungsbeispielen eingerichtet sein, basierend auf dem empfangenen mindestens einen Sensorwert (dem mittels des Mikrofons erfassten Geräusch und der Geschwindigkeit) mindestens eine Information und/oder eine Empfehlung zu ermitteln und einem Nutzer bereitzustellen, indem sie, wie z.B. in FIG. 2A dargestellt, die Empfehlung indirekt ermittelt, beispielsweise wie oben beschrieben, z.B. mittels Übermittelns (veranschaulicht als Übertragungssignal 230) des mindestens einen Sensorwerts an eine externe Datenverarbeitungsvorrichtung 226, z.B. einen externen Computer, z.B. eine Cloud, Vergleichens mit in einem Speicher der externen Datenverarbeitungsvorrichtung 226 gespeicherten Referenzspektren, -werten, -wertebereichen und/oder Daten in einer Datenbank, wobei den Referenzwerten, -wertebereichen bzw. Daten in der Datenbank jeweils mindestens eine Empfehlung zugeordnet sein kann. Die Haarinformations-Ermittlungsvorrichtung 100 kann eingerichtet sein, die von der externen Datenverarbeitungsvorrichtung 226 ermittelte Empfehlung zu empfangen, d.h. die ermittelte Empfehlung kann der Haarinformations-Ermittlungsvorrichtung 100 übermittelt werden (veranschaulicht als Übertragungssignal 224).

In verschiedenen Ausführungsbeispielen kann ein Haarinformations-Ermittlungssystem 200 bereitgestellt sein, wie dies in FIG. 2A, FIG. 2B, FIG. 3A und FIG. 3B beispielhaft gezeigt ist.

Das Haarinformations-Ermittlungssystem kann, wie in FIG. 2A, 2B und 3A dargestellt ist, in verschiedenen Ausführungsbeispielen eine Haarinformations-Ermittlungsvorrichtung 100 gemäß verschiedenen Ausführungsbeispielen und eine Anzeigevorrichtung 228 aufweisen.

Das Haarinformations-Ermittlungssystem kann, wie in FIG. 3B dargestellt ist, in verschiedenen Ausführungsbeispielen eine Haarinformations-Ermittlungsvorrichtung 101 und eine Anzeigevorrichtung 228 aufweisen. Die H-E 101 kann sich von der H-E 100 dadurch unterscheiden, dass sie keinen eigenen Geschwindigkeitssensor-Schaltkreis 110 aufweist, sondern stattdessen eingerichtet ist, beispielsweise mittels einer Halterung, eines Steckers o.ä., fest mit der Anzeigevorrichtung 228, die mit der Datenverarbeitungsvorrichtung 226 integriert ist, beispielsweise in Form eines Smartphones, eines Tablets oder einer ähnlichen Vorrichtung, verbunden zu werden. Dadurch kann ermöglicht werden, anstelle des Geschwindigkeitssensor-Schaltkreises 110 einen in der kombinierten Anzeigevorrichtung/Datenverarbeitungsvorrichtung 226/228 integrierten Sensor für ein Ermitteln der Geschwindigkeit des Vorrichtungskörpers 100K zu verwenden. Mittels der festen Verbindung von H-E 101 und Anzeigevorrichtung/Datenverarbeitungsvorrichtung 226/228 kann sichergestellt sein, dass eine Geschwindigkeit der Anzeigevorrichtung/Datenverarbeitungsvorrichtung 226/228 der Geschwindigkeit des Vorrichtungskörpers 100K entspricht.

Zum Übermitteln des mindestens einen Sensorwerts und/oder der mindestens einen Empfehlung an die Anzeigevorrichtung 228 und/oder zum Übermitteln des mindestens einen Sensorwerts an die externe Datenverarbeitungsvorrichtung 226 und/oder zum Empfangen der mindestens einen Empfehlung von der externen Datenverarbeitungsvorrichtung 226 kann die Haarinformations-Ermittlungsvorrichtung 100/101 mit einer Übertragungsvorrichtung 104a für eine drahtlose Datenübertragung ausgestattet sein, beispielsweise mit einem Chip oder Tag, der die drahtlose Datenübertragung, z.B. mittels Bluetooth, WLAN, ZigBee, NFC, Wibree, Threald, WiMAX oder ähnlichem ermöglicht. In verschiedenen Ausführungsbeispielen kann die Übertragungsvorrichtung 104a Teil der elektronischen Schaltkreisvorrichtung 104 sein.

Wie in FIG. 3A und FIG. 3B dargestellt ist, kann die Anzeigevorrichtung 228 in verschiedenen Ausführungsbeispielen eine integrierte Einheit mit der Datenverarbeitungsvorrichtung 226 bilden, beispielsweise in Form eines Smartphones, Tablets, Laptops, iPads, o.ä.

In verschiedenen Ausführungsbeispielen kann die Haarinformations-Ermittlungsvorrichtung eine integrierte Energieversorgung (nicht dargestellt) aufweisen, beispielsweise wiederaufladbare Batterien o.ä., welche eine Betriebsspannung für die elektronische Schaltkreisvorrichtung 104, die Übertragungsvorrichtung 104a und den mindestens einen Sensor 106 bereitstellen kann. Die Energieversorgung kann in verschiedenen Ausführungsbeispielen auswechselbar gestaltet sein. In verschiedenen Ausführungsbeispielen kann die Energieversorgung (z.B. wasserdicht) verschlossen, z.B. eingegossen, in der Haarinformations-Ermittlungsvorrichtung 100 angeordnet sein. In dem Fall kann die Energieversorgung beispielsweise induktiv aufladbar sein.

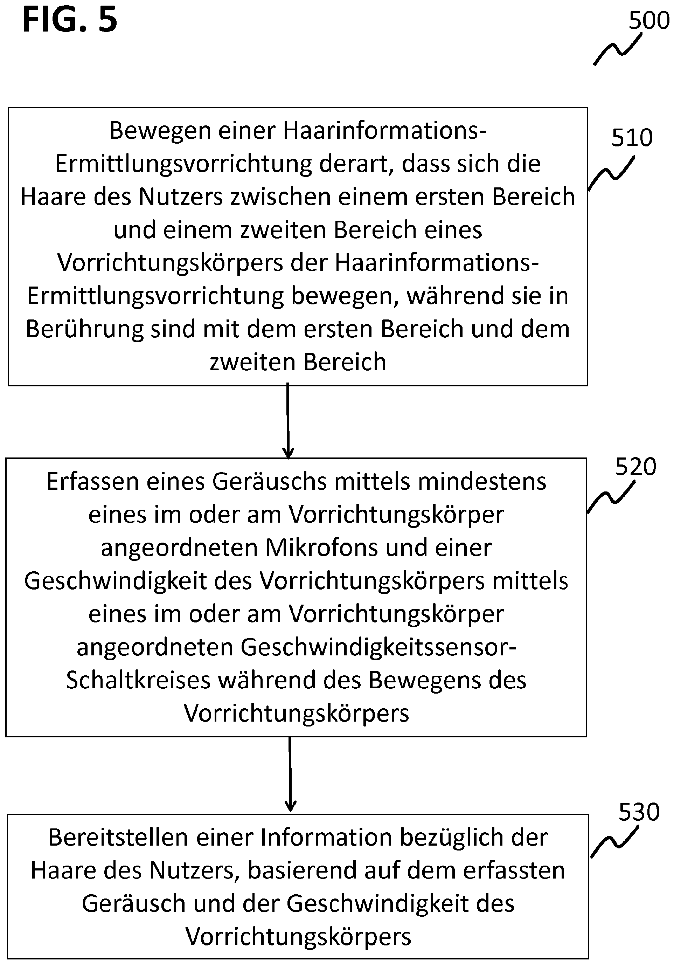

FIG. 5 zeigt ein Flussdiagramm 500 eines Verfahrens zum Bereitstellen einer Haarzustandsinformation gemäß verschiedenen Ausführungsbeispielen.

Bei einem Verfahren zum Bereitstellen einer Haarzustandsinformation gemäß verschiedenen Ausführungsbeispielen kann eine Haarinformations-Ermittlungsvorrichtung gemäß verschiedenen Ausführungsbeispielen und/oder ein Haarinformations-Ermittlungssystem gemäß verschiedenen Ausführungsbeispielen genutzt werden.

Das Verfahren kann aufweisen ein Bewegen einer Haarinformations-Ermittlungsvorrichtung derart, dass sich die Haare des Nutzers zwischen einem ersten Bereich und einem zweiten Bereich eines Vorrichtungskörpers der Haarinformations-Ermittlungsvorrichtung bewegen, während sie in Berührung sind mit dem ersten Bereich und dem zweiten Bereich (in 510), ein Erfassen eines Geräuschs mittels mindestens eines im oder am Vorrichtungskörper angeordneten Mikrofons und einer Geschwindigkeit des Vorrichtungskörpers mittels eines im oder am Vorrichtungskörper angeordneten Geschwindigkeitssensor-Schaltkreises während des Bewegens des Vorrichtungskörpers (in 520) und ein Bereitstellen einer Information bezüglich der Haare des Nutzers, basierend auf dem erfassten Geräusch und der Geschwindigkeit des Vorrichtungskörpers (in 530).

Für die oben beschriebenen Ermittlungen kann in verschiedenen Ausführungsbeispielen eine Programmierung, z.B. eine Software genutzt werden. Dabei kann jede Software genutzt werden, die eine oben beschriebene Funktionalität bereitstellt. In verschiedenen Ausführungsbeispielen, z.B. in einem Fall, dass zum Ausführen des Verfahrens zum Bereitstellen einer Haarzustandsinformation gemäß verschiedenen Ausführungsbeispielen ein Smartphone, ein Tablet oder ähnliches genutzt wird, kann die Software als eine App bereitgestellt sein.

In verschiedenen Ausführungsbeispielen, beispielsweise mittels Vergleichens der Sensorwerte mit bereitgestellten Referenzwerten, können z.B. ein geeignetes Haarpflegemittel, Haarstylingmittel, eine Haarbehandlungsempfehlung oder ähnliches für die Haare ermittelt werden.

In verschiedenen Ausführungsbeispielen kann die in der Haarinformations-Ermittlungsvorrichtung integrierte Schaltkreisvorrichtung und/oder eine externe Datenverarbeitungsvorrichtung, z.B. ein Smartphone, ein Tablet, ein Laptop, ein Smart Mirror, ein iPad, oder ähnliches geeignet sein, um bei einem Ausführen des Verfahrens zum Bereitstellen einer Haarzustandsinformation, z.B. bei Ermittlungsvorgängen, z.B. mittels Vergleichens mit einer Datenbank/Referenzwerten o.ä., verwendet zu werden. In verschiedenen Ausführungsbeispielen braucht die Programmierung/Software nicht auf dem Smartphone, dem Tablet, dem Laptop usw. bereitgestellt zu werden. Es kann beispielsweise ausreichend sein, wenn die in die Haarinformations-Ermittlungsvorrichtung integrierte Schaltkreisvorrichtung und/oder das Smartphone o.ä. durch das Internet, mittels WLAN oder auf andere gängige Weise mit einer (z.B. einer weiteren) externen Datenverarbeitungsvorrichtung, z.B. einem Computer, beispielsweise einer Cloud, verbunden ist. In einem solchen Fall können die Berechnungen beispielsweise mittels der (weiteren) externen Datenverarbeitungsvorrichtung, z.B. mittels des Computers, ausgeführt werden, und das Ergebnis kann dem Smartphone/Tablet o.ä. und/oder der internen Schaltkreisvorrichtung bereitgestellt werden.

Weitere vorteilhafte Ausgestaltungen des Verfahrens ergeben sich aus der Beschreibung der Vorrichtung und umgekehrt.

## Patentansprüche

1. Haarinformations-Ermittlungsvorrichtung (100, 100a) zum Bereitstellen einer Information bezüglich Haaren (220H) eines Nutzers (220), aufweisend:
Einen Vorrichtungskörper (100K) mit mindestens einem ersten Bereich (100K1) und einem zweiten Bereich (100K2), welche so gestaltet sind, dass die Haare (220H) des Nutzers (220) zwischen dem ersten Bereich (100K1) und dem zweiten Bereich (100K2) bewegbar sind, während sie in Berührung sind mit dem ersten Bereich (100K1) und dem zweiten Bereich (100K2);
mindestens ein im oder am Vorrichtungskörper (100K) angeordnetes Mikrofon (106) zum Erfassen eines Geräuschs während eines Bewegens der Haare (220H) zwischen dem ersten Bereich (100K1) und dem zweiten Bereich (100K2);
einen im oder am Vorrichtungskörper (100K) angeordneten Geschwindigkeitssensor-Schaltkreis (110), der mindestens einen Sensor aufweist, zum Ermitteln eines Werts, der eine Geschwindigkeit des Vorrichtungskörpers (100K) repräsentiert; und
eine im oder am Vorrichtungskörper (100K) angeordnete elektronische Schaltkreisvorrichtung (104),
wobei die elektronische Schaltkreisvorrichtung (104) mit dem mindestens einen Mikrofon (106) und dem Geschwindigkeitssensor-Schaltkreis (110) gekoppelt ist zum Empfangen des erfassten Geräuschs und der Geschwindigkeit des Vorrichtungskörpers (100K), und
wobei die elektronische Schaltkreisvorrichtung (104) eingerichtet ist, basierend auf dem empfangenen erfassten Geräusch und der Geschwindigkeit des Vorrichtungskörpers (100K), dem Nutzer (220) eine Information bezüglich seiner Haare (220H) bereitzustellen,
**dadurch gekennzeichnet, dass**
die Information bezüglich der Haare (220H) des Nutzers (220) einen äußeren Haarschädigungsgrad umfasst, wobei ein Frequenzspektrum der erfassten Geräusche in einem Frequenzbereich von 86 Hz bis 22011 Hz mit einer Mehrzahl von Referenz-Frequenzspektren verglichen wird, wobei die Referenz-Frequenzspektren Frequenzspektren von Haaren (220H) aufweisen, deren Haarschädigungsgrad bekannt ist und deren Frequenzspektra mittels einer Datenbank zugeordnet sind, wobei der äußere Haarschädigungsgrad des Nutzers (220) als äußerer Haarschädigungsgrad des ähnlichsten Referenz-Frequenzspektrum zugeordnet wird.

2. Haarinformations-Ermittlungsvorrichtung (100, 100a) gemäß Anspruch 1,
wobei der Vorrichtungskörper (100K) als Kamm oder als Bürste geformt ist, und wobei der erste Bereich (100K1) und der zweite Bereich (100K2) zwei benachbarte Kammzinken oder zwei benachbarte Borsten der Bürste aufweisen.

3. Haarinformations-Ermittlungsvorrichtung (100, 100a) gemäß Anspruch 1 oder 2,
wobei die elektronische Schaltkreisvorrichtung (104) eingerichtet ist, basierend auf dem erfassten Geräusch und der Geschwindigkeit des Vorrichtungskörpers (100K) eine Haarzustandsinformation zu ermitteln und dem Nutzer (220) bereitzustellen.

4. Haarinformations-Ermittlungsvorrichtung (100, 100a) gemäß einem der Ansprüche 1 bis 3,
wobei die elektronische Schaltkreisvorrichtung (104) eingerichtet ist, basierend auf dem erfassten Geräusch und der Geschwindigkeit des Vorrichtungskörpers (100K) eine Empfehlung zu ermitteln und dem Nutzer (220) bereitzustellen.

5. Haarinformations-Ermittlungsvorrichtung (100, 100a) gemäß Anspruch 4, wobei die Empfehlung mindestens eine aufweist von einer Haarpflegeproduktempfehlung, einer Haarstylingproduktempfehlung und einer Haarbehandlungsempfehlung.

6. Haarinformations-Ermittlungsvorrichtung (100, 100a) gemäß einem der Ansprüche 1 bis 4,
wobei das mindestens eine Mikrofon (106) eine Mehrzahl von Mikrofonen aufweist.

7. Haarinformations-Ermittlungsvorrichtung (100, 100a) gemäß einem der Ansprüche 1 bis 6,
wobei das mindestens eine Mikrofon (106) im Vorrichtungskörper (100K) versiegelt angeordnet ist.

8. Haarinformations-Ermittlungsvorrichtung (100, 100a) gemäß einem der Ansprüche 1 bis 7,
wobei die elektronische Schaltkreisvorrichtung (104) eine kabellose Datenaustauschvorrichtung aufweist.

9. Haarinformations-Ermittlungsvorrichtung (100, 100a) gemäß einem der Ansprüche 1 bis 8, ferner aufweisend:
eine Kamera (114) zum Aufnehmen eines digitalen Bildes der Haare (220H).

10. Haarinformations-Ermittlungssystem (200, 200c, 200d), aufweisend:
eine Haarinformations-Ermittlungsvorrichtung (100, 100a) gemäß Anspruch 8 oder 9; und
eine Anzeigevorrichtung (228),
wobei die mindestens eine Haarinformations-Ermittlungsvorrichtung (100, 100a) eingerichtet ist, der Anzeigevorrichtung (228) die Information bezüglich der Haare (220H) des Nutzers (220) mittels der Datenaustauschvorrichtung zu übermitteln.

11. Verfahren zum Bereitstellen einer Information bezüglich Haaren eines Nutzers, aufweisend:
Bewegen einer Haarinformations-Ermittlungsvorrichtung (100, 100a) gemäß einem der Ansprüche 1 bis 9 oder eines Haarinformations-Ermittlungssystems (200, 200c, 200d) gemäß Anspruch 10 derart, dass sich die Haare (220H) des Nutzers (220) zwischen dem ersten Bereich (100K1) und dem zweiten Bereich (100K2) bewegen, während sie in Berührung sind mit dem ersten Bereich (100K1) und dem zweiten Bereich (100K2);
Erfassen eines Geräuschs mittels des mindestens einen Mikrofons (106) und einer Geschwindigkeit des Vorrichtungskörpers (100K) mittels des Geschwindigkeitssensor-Schaltkreises (110) während des Bewegens des Vorrichtungskörpers (100K); und
Bereitstellen einer Information bezüglich der Haare (220H) des Nutzers (20), basierend auf dem erfassten Geräusch und der Geschwindigkeit des Vorrichtungskörpers (100K),
**dadurch gekennzeichnet, dass**
die Information bezüglich der Haare (220H) des Nutzers (220) einen äußeren Haarschädigungsgrad umfasst, wobei ein Frequenzspektrum der erfassten Geräusche in einem Frequenzbereich von 86 Hz bis 22011 Hz mit einer Mehrzahl von Referenz-Frequenzspektren verglichen wird, wobei die Referenz-Frequenzspektren Frequenzspektren von Haaren (220H) aufweisen, deren Haarschädigungsgrad bekannt ist und deren Frequenzspektra mittels einer Datenbank zugeordnet sind, wobei der äußere Haarschädigungsgrad des Nutzers (220) als äußerer Haarschädigungsgrad des ähnlichsten Referenz-Frequenzspektrum zugeordnet wird.

12. Verfahren gemäß Anspruch 11, ferner aufweisend:
Ermitteln mindestens einer Haarzustandsinformation basierend auf dem erfassten Geräusch und der Geschwindigkeit des Vorrichtungskörpers (100K).

13. Verfahren gemäß Anspruch 11 oder 12, ferner aufweisend:
Ermitteln mindestens einer Empfehlung, vorzugsweise einer Haarpflegeproduktempfehlung, einer Haarstylingproduktempfehlung und/oder einer Haarbehandlungsempfehlung, basierend auf dem erfassten Geräusch und der Geschwindigkeit des Vorrichtungskörpers (100K).

14. Verfahren gemäß einem der Ansprüche 11 bis 13, ferner aufweisend:
Übermitteln des erfassten Geräuschs und der erfassten Geschwindigkeit an eine externe Datenverarbeitungsvorrichtung (226); und
Empfangen der von der externen Datenverarbeitungsvorrichtung (226) bereitgestellten Information,
wobei das Ermitteln der mindestens einen Haarzustandsinformation basierend auf dem erfassten Geräusch und der Geschwindigkeit mittels der externen Datenverarbeitungsvorrichtung (226) erfolgt.

## Claims

1. A hair information determination device (100, 100a) for providing information relating to hair (220H) of a user (220), comprising:
a device body (100K) having at least a first region (100K1) and a second region (100K2) which are designed in such a way that the hair (220H) of the user (220) can be moved between the first region (100K1) and the second region (100K2) while in contact with the first region (100K1) and the second region (100K2);
at least one microphone (106) arranged in or on the device body (100K) for detecting a noise during a movement of the hair (220H) between the first region (100K1) and the second region (100K2);
a speed sensor circuit (110) arranged in or on the device body (100K), comprising at least one sensor for determining a value which represents a speed of the device body (100K); and
an electronic circuit device (104) arranged in or on the device body (100K),
the electronic circuit device (104) being coupled to the at least one microphone (106) and the speed sensor circuit (110) for receiving the detected noise and the speed of the device body (100K), and
the electronic circuit device (104) being configured to provide the user (220) with information relating to the hair (220H), based on the received detected noise and the speed of the device body (100K),
**characterized in that**
the information relating to the hair (220H) of the user (220) comprises an external degree of hair damage, a frequency spectrum of the detected noises in a frequency range of 86 Hz to 22011 Hz being compared with a plurality of reference frequency spectra, the reference frequency spectra comprising frequency spectra of hair (220H), the degree of hair damage of which is known and frequency spectra of which are assigned by means of a database, the external degree of hair damage of the user (220) being assigned to an external degree of hair damage of the most similar reference frequency spectrum.

2. The hair information determination device (100, 100a) according to claim 1,
wherein the device body (100K) is in the form of a comb or a brush, and wherein the first region (100K1) and the second region (100K2) comprise two adjacent comb teeth or two adjacent bristles of the brush.

3. The hair information determination device (100, 100a) according to claim 1 or 2,
wherein the electronic circuit device (104) is configured to determine hair condition information on the basis of the detected noise and the speed of the device body (100K) and to provide it to the user (220).

4. The hair information determination device (100, 100a) according to one of claims 1 to 3,
wherein the electronic circuit device (104) is configured to determine a recommendation based on the detected noise and the speed of the device body (100K) and to provide it to the user (220).

5. The hair information determination device (100, 100a) according to claim 4, wherein the recommendation comprises at least one of a hair care product recommendation, a hair styling product recommendation, a hair coloring product and a hair treatment recommendation.

6. The hair information determination device (100, 100a) according to one of claims 1 to 4,
wherein the at least one microphone (106) comprises a plurality of microphones.

7. The hair information determination device (100, 100a) according to one of claims 1 to 6,
wherein the at least one microphone (106) is sealed within the device body (100K).

8. The hair information determination device (100, 100a) according to one of claims 1 to 7,
wherein the electronic circuit device (104) comprises a wireless data exchange device.

9. The hair information determination device (100, 100a) according to one of claims 1 to 8, further comprising:
a camera (114) for recording a digital image of the hair (220H).

10. A hair information determination system (200, 200c, 200d), comprising:
a hair information determination device (100, 100a) according to claim 8 or 9; and
a display device (228),
wherein the at least one hair information determination device (100, 100a) is configured to transmit the information relating to the hair (220H) of the user (220) by means of the data exchange device to the display device (228).

11. A method for providing information relating to hair of a user, comprising:
moving a hair information detection device (100, 100a) according to one of claims 1 to 9 or a hair information determination system (200, 200c, 200d) according to claim 10 in such a way that the hair (220H) of the user (220) moves between the first region (100K1) and the second region (100K2) while in contact with the first region (100K1) and the second region (100K2);
detecting a noise by means of the at least one microphone (106) and a speed of the device body (100K) by means of the speed sensor circuit (110) during movement of the device body (100K); and
providing information relating to the hair (220H) of the user (20) based on the detected noise and the speed of the device body (100K),
**characterized in that**
the information relating to the hair (220H) of the user (220) comprises an external degree of hair damage, a frequency spectrum of the detected noises in a frequency range of 86 Hz to 22011 Hz being compared with a plurality of reference frequency spectra, the reference frequency spectra comprising frequency spectra of hair (220H) of which the degree of hair damage is known and the frequency spectra of which are assigned by means of a database, the external degree of hair damage of the user (220) being assigned to an external degree of hair damage of the most similar reference frequency spectrum.

12. The method according to claim 11, further comprising:
determining at least one piece of hair condition information on the basis of the detected noise and the speed of the device body (100K).

13. The method according to one of claims 11 to 12, further comprising:
determining at least one recommendation, preferably a hair care product recommendation, a hair styling product recommendation, and/or a hair treatment recommendation, on the basis of the detected noise and the speed of the device body (100K).

14. The method according to one of claims 11 to 13, further comprising:
transmitting the detected noise and the detected speed to an external data processing device (226); and
receiving the information which is provided by the external data processing device (226),
wherein the at least one piece of hair condition information is determined by means of the external data processing device (226) on the basis of the detected noise and the speed.

## Revendications

1. Dispositif de détermination d'information capillaire (100, 100a) permettant de fournir une information concernant des cheveux (220H) d'un utilisateur (220), présentant :
un corps de dispositif (100K) comportant au moins une première zone (100K1) et une seconde zone (100K2), lesquelles sont conçues de sorte que les cheveux (220H) de l'utilisateur (220) peuvent être déplacés entre la première zone (100K1) et la seconde zone (100K2) lorsqu'ils sont en contact avec la première zone (100K1) et la seconde zone (100K2) ;
au moins un microphone (106) disposé dans ou sur le corps de dispositif (100K) et permettant de détecter un bruit pendant un déplacement des cheveux (220H) entre la première zone (100K1) et la seconde zone (100K2) ;
un circuit de capteur de vitesse (110) disposé dans ou sur le corps de dispositif (100K), lequel circuit présente au moins un capteur permettant de déterminer une valeur représentant une vitesse du corps de dispositif (100K) ; et
un dispositif à circuit électronique (104) disposé dans ou sur le corps de dispositif (100K),
dans lequel le dispositif à circuit électronique (104) est couplé à l'au moins un microphone (106) et au circuit de capteur de vitesse (110) pour la réception du bruit détecté et de la vitesse du corps de dispositif (100K), et
dans lequel le dispositif à circuit électronique (104) est configuré pour fournir à l'utilisateur (220) une information concernant ses cheveux (220H) sur la base du bruit détecté reçu et de la vitesse du corps de dispositif (100K),
**caractérisé en ce que**
l'information concernant les cheveux (220H) de l'utilisateur (220) comprend un degré d'endommagement des cheveux externe, dans lequel un spectre de fréquences des bruits détectés est comparé à une pluralité de spectres de fréquences de référence dans une plage de fréquences allant de 86 Hz à 22 011 Hz, dans lequel les spectres de fréquences de référence présentent des spectres de fréquences de cheveux (220H) dont le degré d'endommagement des cheveux est connu et dont les spectres de fréquences sont attribués au moyen d'une base de données, dans lequel le degré d'endommagement des cheveux externe de l'utilisateur (220) est attribué comme degré d'endommagement des cheveux externe du spectre de fréquences de référence le plus similaire.

2. Dispositif de détermination d'information capillaire (100, 100a) selon la revendication 1,
dans lequel le corps de dispositif (100K) est formé en tant que peigne ou en tant que brosse, et dans lequel la première zone (100K1) et la seconde zone (100K2) présentent deux dents de peigne adjacentes ou deux poils adjacents de la brosse.

3. Dispositif de détermination d'information capillaire (100, 100a) selon la revendication 1 ou 2,
dans lequel le dispositif à circuit électronique (104) est configuré pour déterminer une information d'état des cheveux sur la base du bruit détecté et de la vitesse du corps de dispositif (100K) et pour la fournir à l'utilisateur (220).

4. Dispositif de détermination d'information capillaire (100, 100a) selon l'une des revendications 1 à 3,
dans lequel le dispositif à circuit électronique (104) est configuré pour déterminer une recommandation sur la base du bruit détecté et de la vitesse du corps de dispositif (100K) et pour la fournir à l'utilisateur (220).

5. Dispositif de détermination d'information capillaire (100, 100a) selon la revendication 4, dans lequel la recommandation présente au moins une recommandation parmi une recommandation de produit de soin des cheveux, une recommandation de produit de coiffage des cheveux et une recommandation de traitement des cheveux.

6. Dispositif de détermination d'information capillaire (100, 100a) selon l'une des revendications 1 à 4,
dans lequel l'au moins un microphone (106) présente une pluralité de microphones.

7. Dispositif de détermination d'information capillaire (100, 100a) selon l'une des revendications 1 à 6,
dans lequel l'au moins un microphone (106) est disposé de manière scellée dans le corps de dispositif (100K).

8. Dispositif de détermination d'information capillaire (100, 100a) selon l'une des revendications 1 à 7,
dans lequel le dispositif à circuit électronique (104) présente un dispositif d'échange de données sans fil (104a).

9. Dispositif de détermination d'information capillaire (100, 100a) selon l'une des revendications 1 à 8, présentant en outre :
une caméra (114) permettant d'enregistrer une image numérique des cheveux (220H).

10. Système de détermination d'information capillaire (200, 200c, 200d), présentant :
un dispositif de détermination d'information capillaire (100, 100a) selon la revendication 8 ou 9 ; et
un dispositif d'affichage (228),
dans lequel l'au moins un dispositif de détermination d'information capillaire (100, 100a) est configuré pour transmettre au dispositif d'affichage (228) l'information concernant les cheveux (220H) de l'utilisateur (220) au moyen du dispositif d'échange de données.

11. Procédé permettant de fournir une information concernant les cheveux d'un utilisateur, présentant :
le déplacement d'un dispositif de détermination d'information capillaire (100, 100a) selon l'une des revendications 1 à 9 ou d'un système de détermination d'information capillaire (200, 200c, 200d) selon la revendication 10, de telle sorte que les cheveux (220H) de l'utilisateur (220) se déplacent entre la première zone (100K1) et la seconde zone (100K2) lorsqu'ils sont en contact avec la première zone (100K1) et la seconde zone (100K2) ;
la détection d'un bruit au moyen de l'au moins un microphone (106) et d'une vitesse du corps de dispositif (100K) au moyen du circuit de capteur de vitesse (110) pendant le déplacement du corps de dispositif (100K) ; et
la fourniture d'une information concernant les cheveux (220H) de l'utilisateur (20) sur la base du bruit détecté et de la vitesse du corps de dispositif (100K), **caractérisé en ce que**
l'information concernant les cheveux (220H) de l'utilisateur (220) comprend un degré d'endommagement des cheveux externe, dans lequel un spectre de fréquences des bruits détectés est comparé à une pluralité de spectres de fréquences de référence dans une plage de fréquences allant de 86 Hz à 22 011 Hz, dans lequel les spectres de fréquences de référence présentent des spectres de fréquences de cheveux (220H) dont le degré d'endommagement des cheveux est connu et dont les spectres de fréquences sont attribués au moyen d'une base de données, dans lequel le degré d'endommagement des cheveux externe de l'utilisateur (220) est attribué comme degré d'endommagement des cheveux externe du spectre de fréquences de référence le plus similaire.

12. Procédé selon la revendication 11, présentant en outre :
la détermination d'au moins une information d'état des cheveux sur la base du bruit détecté et de la vitesse du corps de dispositif (100K).

13. Procédé selon la revendication 11 ou 12, présentant en outre :
la détermination d'au moins une recommandation, de préférence d'une recommandation de produit de soin des cheveux, d'une recommandation de produit de coiffage des cheveux et/ou d'une recommandation de traitement des cheveux, sur la base du bruit détecté et de la vitesse du corps de dispositif (100K).

14. Procédé selon l'une des revendications 11 à 13, présentant en outre :
la transmission du bruit détecté et de la vitesse détectée à un dispositif de traitement de données externe (226) ; et
la réception de l'information fournie par le dispositif de traitement de données externe (226), dans lequel la détermination de l'au moins une information d'état des cheveux est effectuée sur la base du bruit détecté et de la vitesse au moyen du dispositif de traitement de données externe (226).
